# EUROPÄISCHE PATENTSCHRIFT

(11) **EP 3 166 604 B1**
(45) Veröffentlichungstag und Bekanntmachung des Hinweises auf die Patenterteilung: **18.11.2020**
(21) Anmeldenummer: 15756829.6
(22) Anmeldetag: 10.07.2015
(51) Int. Cl.: A61K 31/44, A61P 31/12, C07D 213/73

(54) **MITTEL ZUR BEHANDLUNG RETROVIRALER INFEKTIONSERKRANKUNGEN**
SUBSTANCES FOR THE TREATMENT OF RETROVIRAL INFECTIOUS DISEASES
PRODUIT DESTINÉ AU TRAITEMENT DES MALADIES INFECTIEUSES DUES À DES RÉTROVIRUS

(30) Priorität: 10.07.2014 DE 102014010220
(43) Veröffentlichungstag der Anmeldung: 17.05.2017
(73) Patentinhaber: Immunologik GmbH, 07646 Trockenborn-Wolfersdorf (DE)
(72) Erfinder: SCHUBERT, Ulrich, 07646 Trockenborn-Wolfersdorf (DE); SETZ, Christian, 96117 Memmelsdorf (DE); BRYSCH, Wolfgang, 13505 Berlin (DE); VON WEGERER, Jörg, 13597 Berlin (DE)
(74) Vertreter: Gruber, Daniel
(86) Internationale Anmeldenummer: PCT/DE2015/000357
(87) Internationale Veröffentlichungsnummer: WO 2016/004917

(56) Entgegenhaltungen:
- WO-A1-2008/156676
- WO-A1-2014/100438
- MIKAEL ALTUN ET AL: "Activity-Based Chemical Proteomics Accelerates Inhibitor Development for Deubiquitylating Enzymes", CHEMISTRY & BIOLOGY, Bd. 18, Nr. 11, 1. November 2011 (2011-11-01), Seiten 1401-1412, XP055217598, ISSN: 1074-5521, DOI: 10.1016/j.chembiol.2011.08.018
- MICHAEL R. MATTERN ET AL: "Ubiquitin-based anticancer therapy: Carpet bombing with proteasome inhibitors vs surgical strikes with E1, E2, E3, or DUB inhibitors", BIOCHIMICA ET BIOPHYSICA ACTA (BBA) - MOLECULAR CELL RESEARCH, Bd. 1823, Nr. 11, 1. November 2012 (2012-11-01), Seiten 2014-2021, XP055217597, ISSN: 0167-4889, DOI: 10.1016/j.bbamcr.2012.05.005
- HOLGER A. LINDNER: "Deubiquitination in virus infection", VIROLOGY, Bd. 362, Nr. 2, 1. Juni 2007 (2007-06-01), Seiten 245-256, XP055218247, ISSN: 0042-6822, DOI: 10.1016/j.virol.2006.12.035
- VOGT V M: "Ubiquitin in retrovirus assembly: actor or bystander?", PROCEEDINGS OF THE NATIONAL ACADEMY OF SCIENCES, NATIONAL ACADEMY OF SCIENCES, US, Bd. 97, Nr. 24, 1. Januar 2000 (2000-01-01), Seiten 12945-12947, XP002250795, ISSN: 0027-8424, DOI: 10.1073/PNAS.97.24.12945
- Holger B. Kramer ET AL: "Detection of ubiquitin-proteasome enzymatic activities in cells: Application of activity-based probes to inhibitor development", BIOCHIMICA ET BIOPHYSICA ACTA. MOLECULAR CELL RESEARCH., vol. 1823, no. 11, 1 November 2012 (2012-11-01), pages 2029-2037, XP055509014, NL ISSN: 0167-4889, DOI: 10.1016/j.bbamcr.2012.05.014
- Zejian Liu: "Regulation of monocarboxylate transporter 1 (Mct1) by protein kinase A (PKA), the canonical wnt/beta-catenin, notch signaling pathways, and by a mct metabolic blocker", , December 2013 (2013-12), pages 1-84, ProQuest Dissertations Publishing ISBN: 978-1-339-40454-7 Retrieved from the Internet: URL:https://conservancy.umn.edu/bitstream/ handle/11299/177138/Liu_umn_0130E_14632.pd f?sequence=1&isAllowed=y [retrieved on 2018-09-24]

## Beschreibung

Die vorliegende Erfindung bezieht sich auf neuartige Wirkstoffe zur Behandlung und Prophylaxe retroviraler Infektionserkrankungen. Zudem wird ein neues Wirkprinzip offenbart.

Infektionen mit Retroviren und die daraus entstehenden primären und sekundären Erkrankungen bilden einen bedeutsamen und oftmals bedrohlichen Teil viraler Infektionserkrankungen. Prominentes Beispiel ist die Infektion mit humanen Immundefizienzviren Typ 1 und Typ 2 (HIV-1/HIV-2) und dem dadurch verursachten Ausbruch des erworbenen Immunmangelsyndroms (Acquired Immunodeficiency Syndrome, AIDS) beim Patienten. Auch Tumore wie z.B. Lymphome und Sarkome können durch Retroviren verursacht werden.

Retroviren integrieren ihre Erbinformation in das Genom einer Wirtszelle, um sich mit deren Hilfe zu vermehren. Im Virus liegt das Genom in Form einer einsträngigen RNA vor. Damit dieses sich in das Genom der Wirtszelle integrieren kann, muss es zuvor mit Hilfe des Enzyms Reverse Transkriptase in cDNA umgeschrieben werden. Bisherige Behandlungsansätze bei retroviralen Erkrankungen waren daher stets auf eine Interaktion mit der Reversen Transkriptase (RT) oder den für die Vermehrung zuständigen retroviralen Proteinen wie der Protease (PR) oder Integrase (IN) fokussiert. Durch die vorliegende Erfindung kann gezeigt werden, dass auch regulatorische Proteine der Wirtszelle ein vielversprechendes Target sein können.

Seit Anfang der 1980er Jahre hat die AIDS-Pandemie Millionen von HIV-infizierten Menschen mit einer multisystemischen und bisher unheilbaren Krankheit konfrontiert. Ursprünglich war der Verlauf dieser Erkrankung in den allermeisten Fällen tödlich. Heutzutage steht eine medizinische Behandlungsmöglichkeit zur Verfügung, die Hochaktive antiretrovirale Therapie (HAART; offenbart in WO 00/33654). Diese ermöglicht bei lebenslanger Anwendung - zumindest in den Industriestaaten - das Überleben der Patienten, so dass eine HIV-Infektion bzw. AIDS zu einer chronischen Erkrankung geworden ist. Versagt diese Therapie, ist der Verlauf jedoch weiterhin tödlich. Die HAART ist jedoch teuer und mit gravierenden Nebenwirkungen verbunden. Darüber hinaus ist diese Therapie dadurch limitiert, dass HIV-1 eine enorme, im Vergleich mit der Replikation humaner DNA bis 10⁶-mal höhere Mutationsrate aufweist. Der dadurch bedingte Polymorphismus führt zwangsläufig und in relativ kurzer Zeit zum Auftreten von mutanten HIV-1-Formen, die gegenüber einzelnen und sogar kombinierten anti-HIV-Therapeutika, wie der HAART, resistent sind. Aufgrund dieses hohen Resistenzrisikos, der Therapieresistenz mancher Patienten und der schwerwiegenden Nebenwirkungen besteht daher ein Bedarf, neue Wirkstoffe zur HIV-Therapie zu entwickeln. Daher ist es die Aufgabe der vorliegenden Erfindung, derartige neue Wirkstoffe bereitzustellen. Besonders vorteilhaft wäre es, wenn neue zelluläre Angriffspunkte ("Targets") identifiziert werden könnten.

Die Aufgabe wird gelöst durch die in der allgemeinen Formel (I) beschriebenen Verbindungen. In den Unteransprüchen werden weitere vorteilhafte Ausführungsformen genannt.

Der Humane Immundefizienz-Virus Typ 1 (HIV-1) gehört zur Familie der Retroviren (Gattung: Lentiviridae). Das HIV-1-Genom hat eine Länge von ca. 9 kbp und besitzt drei offene Leserahmen, Gag (group-specific antigens), Pol (PR, RT, Integrase (IN)) und Env (Glykoproteine gp41 und gp120, envelope glycoproteins). Zusätzlich zu diesen bei allen Retroviren kanonisch vorkommenden kodiert HIV-1 für sechs weitere regulatorische Proteine. Tat und Rev sind für die Virusreplikation essentiell. Die akzessorischen Proteine Nef, Vpr, Vif und Vpu sind zwar für die Replikation in Zellkultur nicht zwingend notwendig, spielen aber *in vivo* eine wichtige Rolle.

Die Hauptkomponenten der HIV-Strukturproteine werden in Form von drei Polyproteinen translatiert: Gag und Gag-Pol enthalten die inneren Core-Proteine und viralen Enzyme, wohingegen Env die viralen Hüllproteine umfasst. Membran-Targeting-Signale in der NH₂-terminalen Domäne von Gag sind für den Transport von Gag an die Zellmembran entscheidend. Bei HIV-1 wird das Gag-Polyprotein Pr55 mittels Proteolyse posttranslational in die Matrix (MA), das Capsid (CA), das Nucleocapsid (NC) und das COOH-terminale p6-Protein prozessiert. Nicht-infektiöse Viruspartikel werden von der Plasmamembran abgeschnürt, was als Virus-Budding bezeichnet wird. Unmittelbar nach oder schon während des Buddings beginnt nach Aktivierung der viralen Protease die proteolytische Prozessierung der Gag und Gag-Pol-Polyproteine. Die proteolytische Reifung der Virionen geht mit morphologischen Veränderungen einher. Charakteristisch hierbei ist die Kondensierung des inneren Cores, die in der Ausbildung eines für das reife Virus typischen kegelförmigen Core-Zylinders resultiert.

Proteasomen sind multikatalytische Enzymkomplexe, die ca. 1% des Gesamt-Zellproteins ausmachen und die hauptsächliche proteolytische Komponente im Zellkern und Zytosol eukaryontischer Zellen bilden. Proteasomen übernehmen viele wichtige Funktionen im Zellmetabolismus. Die hauptsächliche Funktion ist die Proteolyse von missgefalteten, nichtfunktionellen Proteinen. Eine weitere wichtige Funktion ist der Abbau von zellulären und viralen Proteinen für die T-Zell-vermittelte Immunantwort und dadurch die Generierung von Peptid-Liganden, die auf MHC-I-Moleküle (MHC = major histocompatibility complex) geladen werden. Eine Unterform stellt das Immunoproteasom dar, das in bestimmten Zelltypen, wie z.B. in der Milz, Lymphknoten und in Antigen- präsentierenden Zellen konstitutiv exprimiert wird.

Substrate der Proteasomen werden in der Regel durch die Anheftung von Ubiquitin-Oligomeren für den Abbau markiert. Ubiquitin (Ub) ist ein hochkonserviertes, 76 Aminosäuren langes Protein, das kovalent an das jeweilige Zielprotein gekoppelt wird. Die Ubiquitinylierung stellt einen reversiblen Prozess dar. Die Ub-Moleküle können durch eine Vielzahl an DUBs (deubiquitinating enzymes) wieder von dem Zielmolekül entfernt werden. Damit stehen die Ub-Moleküle wieder intrazellulär zur Verfügung. Dieser Recycling-Vorgang ist für die Homöostase der Zelle von entscheidender Bedeutung. Dieses regulatorische System aus Ubiquitinylierung von Target-Proteinen und der proteasomalen Proteolyse wird üblicherweise als Ubiquitin-Proteasom-System (UPS) bezeichnet.

Die DUBs sind eine umfangreiche Klasse an Ub-Hydrolasen und sind intrazellulär der Gegenspieler der Ubiquitin-E3-Ligasen. Die Zielproteine können durch die DUBs vollständig oder teilweise deubiquitinyliert werden. Beim Menschen sind bislang 90 DUBs bekannt, welche in fünf Familien unterteilt werden:
- ubiquitin-specific protease family (USP)
- ubiquitin C-terminal hydrolases (UCHs)
- ovarian tumour proteases (OTUs)
- Josephin family
- JAB1/MPN/Mov34 family (JAMMs)

Die ersten vier zählen zu den Cystein-Proteasen, während es sich bei der letzten um eine Zink-Metalloprotease handelt.

Die wichtigsten zellulären Funktionen der DUBs sind:
1. Sie sind für die neue Generierung von freiem Ubiquitin von entscheidender Bedeutung. Ubiquitin ist ein auf mehreren Genen kodiertes lineares Fusionsprotein aus einer Reihe von Ub-Monomeren. Nach der Translation wird diese Ubiquitin-Kette spezifisch von DUBs hydrolisiert, so dass es zur Freisetzung freier Ub-Moleküle kommt.
2. Sie entfernen hochspezifisch Polyubiquitin-Ketten von posttranslational modifizierten Proteinen. Hierdurch wird das Zielprotein stabilisiert. Des Weiteren entfernen die mit dem Proteasom assoziierten DUBs POH1, UCH37 und USP14 Ubiquitin-Ketten von Proteinen, die bereits zur Proteolyse in das Proteasom eingetreten sind. Auf diese Weise wird der Gehalt an freiem Ubiquitin in der Zelle im Gleichgewicht gehalten.
3. Sie verändern Ubiquitin-Modifikationen von Proteinen, indem die bestehenden Ubiquitin-Ketten getrimmt werden. So kann beispielsweise ein Protein, das ursprünglich polyubiquitinyliert wurde, am Ende nur noch ein einziges Ubiquitin tragen. Die Funktion des Proteins kann dadurch vollständig unterschiedlich sein.

DUBs weisen meistens eine hohe Spezifität für einzelne Substrate als auch für bestimmte Ubiquitin-Ketten auf.

DUBs unterliegen wiederum selbst einer komplexen Regulierung. So können posttranslationale Modifikationen wie z.B. Phosphorylierung, Ubiquitinylierung oder Sumoylierung auftreten, die zur Aktivierung oder Deaktivierung des jeweiligen DUBs führen können.

Auch kann es bei DUBs durch Bindung an bestimmte Proteine zu einer Konformationsänderung kommen. Hieraus kann ebenfalls eine Aktivierung oder Deaktivierung des jeweiligen DUBs resultieren.

Einige DUBs sind in ihrer Aktivität auf bestimmte Zellkompartimente beschränkt. Bei Bedarf werden sie dorthin transportiert.

Somit bilden die DUBs ein interessantes Target, um durch ihre Inhibierung oder die Modifikation ihrer Aktivität auf zelluläre Regulationsprozesse einzuwirken. Auch für klinische Anwendungen sind sie wie das gesamte UPS in den Fokus des Interesses gerückt. So wurde beispielsweise in den letzten Jahren versucht, mit der Hilfe von *small molecules* Komponenten des UPS wie z.B. das 26S-Proteasom oder Ub-Ligasen zu inhibieren. Man versprach sich dadurch vor allem neue Ansätze für Behandlungsmöglichkeiten bei Tumoren. Das einzige bisher zur Therapie zugelassene Medikament ist der Proteasom-Inhibitor Bortezomib (Velcade®) zur Behandlung von Myelomen und T-Zell-Lymphomen. Der Wirkmechanismus ist allerdings nicht spezifisch. Die globale Wirkung betrifft zahlreiche vitale Prozesse, was teilweise gravierende Nebenwirkungen zur Folge hat, wie z.B. die Ausbildung einer peripheren Neuropathie mit Schmerzen und Taubheitsgefühlen vor allem in den Extremitäten. Bei der klinischen Entwicklung von Inhibitoren der E3-Ligasen ist bisher noch kein Durchbruch gelungen.

Auch bei der Forschung zu DUB-Inhibitoren steht die Entwicklung neuer Therapieansätze in der Tumorbehandlung im Mittelpunkt des Interesses. Die bisher bekannten DUB-Inhibitoren wirken teilweise sehr spezifisch auf ein bestimmtes DUB-Target. Dadurch weisen sie eine recht geringe Zytotoxizität auf. Dies lässt deutlich weniger und mildere Nebenwirkungen im Patienten erwarten als bei einer Anwendung von Inhibitoren des 26S-Proteasoms oder von E1-Ligasen.

Für einige DUBs wurden onkogene Eigenschaften nachgewiesen, so bei USP2a, USP7, USP20 und USP33. Daher wird die Inhibierung von DUBs als Wirkprinzip als Methode angesehen, um die onkogenen Eigenschaften dieser DUBs zu blockieren oder zumindest zu reduzieren.

Der DUB-Inhibitor WP1130 bewirkt eine Herunter-Regulierung von anti-apoptotischen Genen und eine Hoch-Regulierung von pro-apoptotischen Proteinen, wie z.B. von MCL-1 und p53. Dies induziert eine Apoptose in den getesteten Tumorzelllinien (Bartholomeusz et al., 2007, Blood 109(8), 3470-3478; Kapuria et al., 2010, Cancer Res 70(22), 9265-9276).

In CB-17-Mäusen führte die Verwendung des DUB-Inhibitors P005091 zur Apoptose von multiplen Myelom-Zellen. Eine Resistenzbildung unter Bortezomib konnte auf diese Weise revertiert werden (Chauhan et al., 2012, Cancer Cell 22(3), 345-358).

b-AP15 (ein für UCH37/UCH-L5 und USP14 spezifischer DUB-Inhibitor) vermag in verschiedenen *in vivo* Tumormodellen in der Maus die Tumorprogression zu inhibieren. Hierbei wird eine tägliche Injektion von 5 mg/kg Körpergewicht von den Mäusen toleriert (D'Arcy et al., 2011, Nat Med 17(12), 1636-1640).

2012 konnte gezeigt werden, dass man *in vitro* mit dem DUB-Inhibitor WP1130 die Replikation von Noro-, Enzephalomyocarditis- und Sindbis-Virus hemmen kann (Perry et al., 2012, PLoS Pathog 8(7):e1002783).

In der WO 2008/156676 wird der DUB-Inhibitor PR-619 in einer Reihe von Substanzen genannt, die in der Lage sind, die O-Glykolisierung von Proteinen zu inhibieren. Dadurch sollen Krankheiten, bei denen O-Glykolisierung auftritt, behandelt werden, wie z.B. Morbus Alzheimer, Krebs, Diabetes mellitus und Folgekrankheiten, Insulinresistenz, Tumorgenese Metastasenbildung, bakterielle Infektionen und ihrer Folge Sepsis.

In Vogt (2000, PNAS 97: 12945-12947) wird beschrieben, dass zelluläres Ubiquitin mit viralen Proteinen beim Assembly von Retroviren wie HIV-1 und RSV (Rous-Sarcoma-Virus) interagiert. DUB-Inhibitoren oder PR-619 zur therapeutischen Intervention werden in diesem Artikel nicht genannt.

In der WO 2014/100438 werden Histondeacetylase-Inhibitoren offenbart, die alleine oder in Kombination mit anderen Wirkstoffen in der Behandlung einer Vielzahl an Krankheiten verwendet werden sollen, u.a. solchen, die in der Folge von AIDS auftreten können wie HIVinduzierter Demenz, fungalen opportunistischen Infektionen und AIDS-induzierten Lymphomen. Es wird in diesem Zusammenhang eine Kombination mit einem Proteasom-Inhibitor oder dem DUB-Inhibitor PR-619 offenbart, nicht jedoch deren alleinige oder kombinierte Verwendung.

Die in obiger PLoS Pathog 2012-Veröffentlichung untersuchten Virusfamilien unterscheiden sich signifikant von der Familie der Retroviren. Noroviren gehören zur Familie der Caliciviridae, Enzephalomyocarditis-Viren gehören zur Familie der Picornaviridae und Sindbis-Viren gehören zur Gattung der Alphaviridae. Diese Virusfamilien lösen nach Infektion beim Menschen keine Immundefizienz aus. Im Gegensatz zu einer Infektion mit Retroviren, erfolgt keine Integration des Virusgenoms in das Wirtsgenom. Weiterhin unterscheidet sich der pathologische Mechanismus dieser Viren deutlich von dem der Retroviren. Der medizinische Bedarf (medical need) ist bei diesen Infektionen deutlich geringer als bei Retroviren. Deshalb ist keine medikamentöse Therapie entwickelt worden, welche die Replikation von Noro-, Enzephalomyocarditis- und Sindbis-Viren hemmt.

Noroviren verursachen beim Menschen meist eine virale Gastroenteritis, welche jedoch in den meisten Fällen nach drei Tagen abklingt und meist komplikationslos verläuft. Enzephalomyocarditis-Viren sind nicht humanpathogen. Sie lösen im Menschen keine Erkrankung aus. Sowohl bei Noroviren als auch Enzephalomyocarditis-Viren handelt es sich, im Gegensatz zu Retroviren, um nicht umhüllte RNA-Viren. Bei der Infektion mit Sindbis-Viren kann in vereinzelten Fällen eine meist harmlose fiebrige Erkrankung mit Entzündungen der Gelenke auftreten, welche schnell wieder abklingt. Im Gegensatz dazu führen die Infektion mit HIV-1 und die daraus resultierende Immundefizienz ohne antiretrovirale Behandlung mit ART fast immer zum Tod.

Überraschenderweise konnte gezeigt werden, dass die erfindungsgemäßen Verbindungen gemäß der allgemeinen Formel (I) in der Lage sind, späte Prozesse im Replikationszyklus von Retroviren zu hemmen. 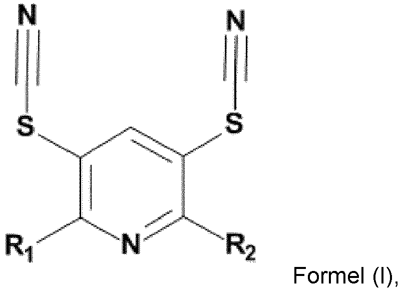 wobei
R₁ und R₂ jeweils unabhängig voneinander für H, -OH, -NH₂, -NHR₃, -NR₃R₄, einen substituierten oder nicht-substituierten, linearen oder verzweigten Alkyl-Rest mit 1 bis 3 Kohlenstoffatomen, -CO-OCH₃, -CO-OC₂H₅, -CO-NH₂, -NO₂, -Cl, -Br, -F, -SO₂H stehen;
R₃ und R₄ jeweils unabhängig voneinander für -OH, -CH₃, -C₂H₅, -CH₂OH, -CHO, -COOH, - CO-CH3, -CO-NH₂ stehen.

Bevorzugt wird, wenn R₁ und R₂ jeweils unabhängig voneinander für H, -OH, -NH₂, -NO₂, - Cl, -F, -SO₂H stehen.

In einer besonders bevorzugten Ausführungsform stehen R₁ und R₂ jeweils für -NH₂.

Eine besonders bevorzugte Ausführungsform ist unter den Namen 2,6-Diaminopyridin-3,5-bis(thiocyanat) oder PR-619 bekannt (Formel II). Da die internationale Nomenklatur nicht einheitlich ist, ist PR-619 auch unter dem Namen 3,5-Dithiocyanatopyridin-2,6-diamin bekannt. Im Sinne dieser Anmeldung sollen alle drei Namen synonym behandelt werden und sich auf dieselbe Verbindung beziehen.

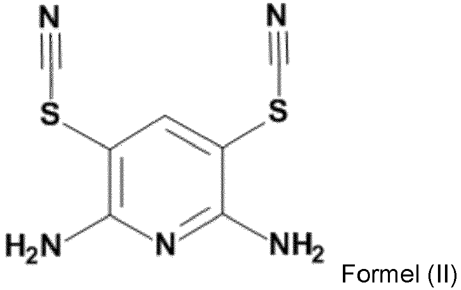

Die Erfindung bezieht sich ebenfalls auf alle Salze, Hydrate und Solvate sowie die Salze der Hydrate und Solvate der erfindungsgemäßen Verbindungen. Geeignete Salze werden weiter unten genannt.

Eine Synthese-Anleitung für PR-619 und verwandte Substanzen findet sich in Beer et al. 2002, J. Am. Chem. Soc. 124, p. 9498-9509.

Aufgrund weiterer Untersuchungen der Anmelderin und vergleichenden Strukturanalysen ist davon auszugehen, dass die entscheidende Struktur für das Wirkprinzip von PR-619 in dem Dithiocyanatopyridin-Gerüst wie in der allgemeinen Formel (I) beschrieben liegt. Daher kann vernünftigerweise davon ausgegangen werden, dass moderat substituierte Derivate von PR-619 einen vergleichbaren Effekt im Sinne der Erfindung erzielen. Dies drückt sich in der allgemeinen Formel (I) und ihren variablen Resten aus.

PR-619 ist bisher als neuartiger DUB-Inhibitor bekannt. Diese Verbindung hemmt USP1, USP2, USP4, USP5, USP7, USP8, USP9X, USP10, USP14, USP15, USP16, USP19, USP20, USP22, USP24, USP28, USP47, USP48, UCH-L1, UCH-L3, UCH-L5/UCH37, ATXN3, BAP1, JOSD2, OTUD5, VCIP135 und YOD. Die Applikation von PR-619 führt zu einer Anreicherung von Polyubiquitin-Ketten (Altun et al., 2011, Chem Biol 18(11), 1401-1412). Auch für einen weiteren DUB-Inhibitor, P22077, konnte eine Aktivierung des Autophagie-Pathways gezeigt werden (Seiberlich et al., 2013, Cell Biochem Biophys 67(1), 149-160). Bisher hat sich die Erforschung von PR-619 auf die Erforschung der Behandlung von Tumoren konzentriert, ohne dass aber für PR-619 ein therapeutisch nutzbares antiproliferatives Potential gefunden werden konnte.

In der vorliegenden Anmeldung konnte gezeigt werden, dass PR-619 und seine Derivate effektiv die proteolytische Prozessierung der Gag-Polyproteine durch die HIV-1-Protease unterbinden können (siehe Ausführungsbeispiel 1). In Folge dieser Inhibition werden die Replikation und damit die Infektionsausbreitung des HI-Virus gehemmt. Wie zuvor beschrieben, ist die proteolytische Prozessierung von Gag ein essentieller Schritt in der Replikation von Retroviren. Wird dieser Schritt inhibiert, wird die Vermehrung der Viren effektiv gehemmt (siehe Ausführungsbeispiele 2 und 10). Somit kann die Virusinfektion therapiert oder die Virenlast zumindest stark eingeschränkt werden, so dass das körpereigene Immunsystem deutlich besser in der Lage ist, die virale Infektion zu kontrollieren und zu bekämpfen.

Dieser Mechanismus der proteolytischen Prozessierung von Gag ist allen Retroviren gemeinsam. Somit sind die erfindungsgemäßen Verbindungen in der Lage, nicht nur die Replikation von HIV-1 zu hemmen, sondern auch die aller anderen bekannten Retroviren, insbesondere bei den mit HIV-1 besonders verwandten Lentiviren, HIV-2, Felinem Immundefizienz-Virus (FIV) und Simianem Immundefizienz-Virus (SIV).

Daher wird die Behandlung und Prophylaxe von Infektionen mit HIV-1, HIV-2, FIV und SIV sowie AIDS mit einer erfindungsgemäßen Verbindung besonders bevorzugt. Am meisten bevorzugt ist die Behandlung und Prophylaxe von Infektionen mit HIV-1 sowie AIDS.

In Ausführungsbeispiel 3 wurde der beschriebene inhibitorische Effekt von PR-619 mit weiteren kommerziell erhältlichen DUB-Inhibitoren verglichen. Das Versuchsdesign war dasselbe wie in Beispiel 1. P005091 (1-[5-[(2,3-Dichlorophenyl)thio]-4-nitro-2-thienyl]ethanon) hemmt spezifisch USP7. Es wurde beschrieben, dass P005091 Apoptose in Tumorzelllinien induziert (Chauhan et al., 2012, Cancer Cell 22(3), 345-358). WP1130 ((2E)-3-(6-Bromo-2-pyridinyl)-2-cyano-N-[(1S)-1-phenylbutyl]-2-propenamid) hemmt spezifisch USP9, USP5, USP14 und UCH37. WP1130 führt zur Akkumulation von polyubiquitinyliertem p53, JAK2 und Bcr-Abl, und reduziert das Proteinlevel von c-Myc und MCL-1 in Tumorzellen (Bartholomeusz et al., 2007, Blood 109(8), 3470-3478; Kapuria et al., 2010, Cancer Res 70(22), 9265-9276).

Es konnte gezeigt werden, dass P005091 und WP1130 keinen Einfluss auf die Replikation des HI-Virus haben. P22077 jedoch zeigt einen zu PR-619 vergleichbaren Effekt, es hemmt ebenfalls effektiv die HIV-1-Replikation und damit die Virusausbreitung.

Wie zuvor ausgeführt, wird der Effekt von PR-619 über die Hemmung eines DUB-Pathways bewirkt. PR-619 wirkt auf eine ganze Reihe von DUBs inhibierend (s.o.). Es ist davon auszugehen, dass PR-619 über dasselbe DUB-Target wirkt wie P22077. P22077 ist jedoch spezifisch für USP7 und USP47. PR-619 hemmt u.a. ebenfalls USP7 und USP47. Das nicht effektive P005091 hemmt jedoch spezifisch USP7. Weder P005091 noch WP1130 hemmen USP47. Daraus folgt zwangsläufig, dass die hemmende Wirkung von PR-619 auf die HIV-1-Replikation über eine Inhibition von USP47 erfolgt. Dies wird durch die Ergebnisse mit P22077 bestätigt. Da die zugrundeliegende proteolytische Prozessierung des Gag-Proteins in dieser Form bei allen Retroviren stattfindet, muss dieses Wirkprinzip über eine Inhibition von USP47 bei der Behandlung und Prophylaxe aller retroviralen Infektionen therapeutisch wirksam sein.

Für eine derartige spezifische Wirkung auf USP47 spricht auch, dass sich USP7 und USP47 erheblich in ihrer subzellulären Lokalisation und damit auch in ihrer Funktion unterscheiden. Während USP7 nur im Zellkern zu finden ist, ist USP47 ausschließlich im Zytosol lokalisiert. USP7 interagiert hauptsächlich mit einer Reihe von Tumorproteinen und reguliert zelluläre Transkriptionsfaktoren. Weiterhin konnte gezeigt werden, dass USP7 in der Adipogenese eine wichtige Rolle spielt (Gao et al., 2013, Nat Commun 4, 2656). USP7 interagiert nur mit Proteinen, welche im Zellkern lokalisiert sind oder dorthin transportiert werden (Nicholson and Kumar, 2011, Cell Biochem Biophys 60, 61-68). Für USP47 konnte gezeigt werden, dass es mit der Ub-E3-Ligase β-TRCP interagiert und dadurch das Zellwachstum und das Überleben der Zelle reguliert, und dass es bei DNA-Reparaturmechanismen eine wichtige Rolle spielt (Parsons et al., 2011, Mol C-ell 41, 609-615; Peschiaroli et al., 2010, Oncogene 29, 1384-1393).

Da die Virusassemblierung von HIV-1 nicht im Zellkern stattfindet, USP7 jedoch ausschließlich dort lokalisiert ist, kann davon ausgegangen werden, dass USP7 hierbei keine Rolle spielt. Somit ist die erfindungsgemäße Hemmung der HIV-1 Replikation mittels des DUB-Inhibitors P22077, welcher sowohl USP7 als auch USP47 hemmt, spezifisch auf die Inhibition von USP47 zurückzuführen. Darüber hinaus konnten wir zeigen, dass der DUB-Inhibitor P005091, welcher spezifisch nur USP7 inhibiert, die HIV-1 Replikation nicht hemmen kann.

Daher bezieht sich die vorliegende , Offenbarung auch auf ein Verfahren zur Behandlung und Prophylaxe von retroviralen Infektionen durch die Verabreichung eines USP47-Inhibitors. Bevorzugt wird eine therapeutische Anwendung dieses Verfahrens zur Behandlung von HIV-1-Infektionen sowie zur Behandlung und Prophylaxe von AIDS.

In den Ausführungsbeispielen 4 und 11 konnte gezeigt werden, dass die zuvor gezeigten Effekte von PR-619 nicht auf unspezifischen zytotoxischen Effekten beruhen. Über einen Behandlungszeitraum von 15 Tagen weist PR-619 keine wesentliche Verringerung des Prozentsatzes überlebender Zellen auf. Lediglich bei einer gegenüber der effektiven Dosis von 3,5 µM wesentlich höheren Dosis (28 µM) treten toxische Effekte auf.

Der Vergleich mit den DUB-Inhibitoren P005091 und WP1130 ergab, dass bei P005091 keine zytotoxischen Effekte zu beobachten waren. Bei WP1130 hingegen zeigte sich bei permanenter Behandlung bereits in der niedrigsten eingesetzten Konzentration von 3 µM ein Rückgang der lebenden Zellen um 40%.

Des Weiteren wurde gefunden, dass die erfindungsgemäße Verwendung von PR-619 die Immunogenität von HIV-1-Strukturproteinen steigert. Dieses Phänomen wird sowohl in HIV-1-infizierten primären PBMC-Kulturen als auch in Kulturen von humanen Fibroblasten (HeLa-Zellen), die mit viraler DNA transfiziert wurden, beobachtet. Dies wurde mit immunologischen Methoden gezeigt. Die Behandlung von HeLa-Zellen mit PR-619 führt zu einer dosisabhängigen Erhöhung der MHC-I-Antigenpräsentation von Epitopen, welche von HIV-1-Strukturproteinen abstammen (siehe Aufführungsbeispiel 5). Diese erhöhte MHC-I-Antigenpräsentation führt auch zu einer verstärkten Aktivierung von CD8⁺-T-Zellen, was die Eliminierung von infizierten Zellen durch zellvermittelte Immunität erhöht. Dadurch könnten *in vivo* spezifisch mit HIV-1 infizierte Zellen schneller erkannt und effizient zerstört werden. Somit lässt sich feststellen, dass PR-619 und seine Derivate dosisabhängig in der Lage sind,
a) die proteolytische Prozessierung der Gag-Polyproteine durch die HIV-1-Protease zu blockieren bzw. zu reduzieren;
b) die Infektiösität der freigesetzten Virionen zu blockieren bzw. zu reduzieren; und
c) die Immunogenität von viralen Strukturproteinen zu erhöhen und damit verbunden eine stark verbesserte zytotoxische CD8⁺-T-Zell-Antwort auszulösen.

Damit sind PR-619 und seine Derivate geeignete Verbindungen zur therapeutischen Behandlung von retroviralen Infektionen, insbesondere von HIV-1-Infektionen, sowie zur Prophylaxe und Behandlung von AIDS.

Weitere besondere Vorteile der Verwendung von PR-619 und seinen Derivaten liegen in der vergleichsweise geringen Zytotoxizität, was eine effektive Dosierung des Wirkstoffes ermöglicht und geringe bzw. milde Nebenwirkungen im Patienten vermuten lässt. Besonders vorteilhaft ist zudem, dass durch das allen Retroviren gemeinsame Wirkprinzip über die Hemmung der proteolytischen Prozessierung des Gag-Polyproteins die Verwendung eines einzigen Wirkstoffs eine ganze Bandbreite retroviraler Infektionen bekämpfen kann. Dies ist von besonderer Bedeutung bei der Behandlung seltener retroviraler Infektionen, für die sich aufgrund der geringen Fallzahl und/oder des geringen kommerziellen Potentials die Entwicklung eines eigenständigen antiretroviralen Wirkstoffes nicht als lohnend darstellen würde. Zudem könnten damit therapeutisch und prophylaktisch Infektionen mit neuartigen Retroviren behandelt werden. Auch bei unklarer Diagnose, was für eine retrovirale Infektion genau vorliegt, erscheint eine Anwendung der erfindungsgemäßen Verbindungen vielversprechend.

Vorteilhaft ist zudem, dass mit den Verbindungen der Formel (I) eine strukturell deutlich andere Alternative zu der HAART bereitgestellt wird. Dies ist insbesondere bei der Resistenzproblematik bei HIV von besonderer Bedeutung.

Die bisher bekannten antiretroviralen Wirkstoffe (z.B. aus der HAART) greifen alle an den viralen Enzymen an, v.a. an der Reversen Transkriptase und der Protease. Die erfindungsgemäßen Verbindungen blockieren jedoch spezifisch einen Mechanismus der Wirtszelle, ohne diese zytotoxisch zu schädigen. Bisher haben sich die Retroviren, insbesondere das HI-Virus aufgrund seiner hohen Mutationsrate vergleichsweise schnell an die angreifenden Wirkstoffe "anpassen" können und Resistenzen entwickelt. Dadurch kommt es über die Zeit zu immer mehr therapierefraktären retroviralen Infektionen. Beim HI-Virus treten Mutationen bis zu 10⁶mal häufiger auf als beim Menschen. Somit versprechen die erfindungsgemäßen Substanzen einen vielfach längeren Anwendungszeitraum als die klassischen anti-retroviralen Medikamente. Das Ubiquitin-Proteasom-System als ein wichtiger intrazellulärer Regulationsmechanismus ist in der Evolution relativ stark konserviert. Hier sind erfolgreiche Mutationen nicht sehr wahrscheinlich, da der Wirtsorganismus (der Mensch) keinen evolutionären Vorteil aus einer solchen Mutation ziehen könnte. Im Gegenteil wird der Wirtsorganismus von der Belastung durch eine retrovirale Infektion befreit oder zumindest stark erleichtert.

Somit bezieht sich die vorliegende Anmeldung auf Verbindungen gemäß Formel (I) zur Verwendung in der Behandlung und/oder Prophylaxe retroviraler Infektionen. Dies schließt die pharmazeutisch verträglichen Salze, Hydrate und Solvate der Substanzen ein.

Die Anmeldung bezieht sich gleichermaßen auf die Verwendung einer Verbindung gemäß Formel (I) zur Herstellung einer pharmazeutischen Zusammensetzung zur Behandlung und/oder Prophylaxe retroviraler Infektionen.

Besonders bevorzugt wird die Verwendung von PR-619 in der Medizin im Allgemeinen und im Besonderen zur Herstellung einer pharmazeutischen Zusammensetzung zur Behandlung und/oder Prophylaxe retroviraler Infektionen.

Allen bekannten Retroviren ist dieselbe Form der proteolytischen Prozessierung der Gag-Strukturproteine gemein. Durch die Verwendung der DUB-Inhibitoren im Sinne der Erfindung wird daher derselbe Wirkmechanismus bei allen Retroviren aktiviert, so dass deren Replikation erfolgreich unterbunden werden kann. Daher sind die Verbindungen im Sinne der Erfindung geeignet, therapeutisch und prophylaktisch bei Retroviren-Infektionen und davon herrührenden Sekundärerkrankungen eingesetzt zu werden. Die erfindungsgemäßen Substanzen sind über ihren Wirkmechanismus in der Lage, die Ausbreitung der Retroviren im Wirtsorganismus zu unterbinden oder zumindest stark abzuschwächen (siehe Ausführungsbeispiele). Damit kann die systemische Ausbreitung einer retroviralen Infektion im Organismus verhindert werden. Auf diese Weise können die erfindungsgemäßen Substanzen auch zur Prophylaxe verwendet werden.

Im Sinne der vorliegenden Anmeldung soll der Begriff HIV oder HI-Virus alle bekannten Gruppen und Subtypen umfassen, insbesondere Gruppe M mit den Subtypen A (A1 - A4), B, C, D, E, F (F1 - F2), G, H, I, J, K und den CRFs (circulating recombinant forms), sowie die Gruppen N, O und P.

HIV-2, Feline Immundefizienz-Virus (FIV) und Simiane Immundefizienz-Virus (SIV) sind mit HIV-1 strukturverwandt.

Eine Infektion mit HIV-1 oder HIV-2 führt in den allermeisten Fällen zu einem Ausbruch von AIDS, wenn der Patient nicht in geeigneter Form, z.B. durch HAART, therapiert wird. AIDS wiederum zieht oftmals eine ganze Reihe von Folgekrankheiten nach sich.

Somit bezieht sich die vorliegende Erfindung ebenfalls auf die Verwendung einer Verbindung gemäß Formel (I) zur Herstellung einer pharmazeutischen Zusammensetzung zur Behandlung und/oder Prophylaxe von HIV-1-Infektion, HIV-2-Infektion, AIDS, FIV-Infektion und SIV-Infektion.

Besonders bevorzugt wird die Verwendung von PR-619 zur Herstellung einer pharmazeutischen Zusammensetzung zur Behandlung und/oder Prophylaxe von HIV-1-Infektion, HIV-2-Infektion, AIDS, FIV-Infektion und SIV-Infektion.

Des Weiteren ist eine therapeutische oder prophylaktische Verwendung der Verbindungen der Formel (I) bei folgenden weiteren Retroviren-Gruppen möglich: Spumaviren, insbesondere Simianes Foamy-Virus (SFV) und Bovines Foamy-Virus (BFV); Alpha-Retroviren, insbesondere Rous Sarcoma-Virus (RSV) und Aviäres Leukose-Virus; Beta-Retroviren, insbesondere Mouse mammary tumor virus (MMTV) und Mason-Pfizer monkey virus (MPMV); Gamma-Retroviren, insbesondere Murines Leukämie-Virus (MLV) und Felines Leukämie-Virus (FeLV); Delta-Retroviren, insbesondere Bovines Leukämie-Virus (BLV) und Humanes T-lymphotropes Virus (HTLV); Epsilon-Retroviren, insbesondere Walleye dermal sarcoma virus (WDSV) und Walleye epidermal hyperplasia virus (WEHV). Verbindungen gemäß der allgemeinen Formel (I) sind auch geeignet, zusammen mit mindestens einem weiteren Wirkstoff verwendet zu werden, wobei der besagte weitere Wirkstoff aus einer Gruppe umfassend Reverse Transkriptase-Inhibitoren, Integrase-Inhibitoren, HIV-Protease-Inhibitoren, Entry-Inhibitoren, HIV-Vakzine, Virostatika und Immunstimulanzien ausgewählt wird. Auch kann eine Kombination aus mindestens einer erfindungsgemäßen Verbindung und mindestens einem anderen Inhibitor zellulärer Faktoren, wie z.B. einem Proteasom-Inhibitor (PI) verwendet werden. Studien zur Wirkung von HIV mit Proteasom-Inhibitoren werden bereits durchgeführt. Überraschenderweise wurde festgestellt, dass eine kombinierte Applikation des Proteasom-Inhibitors Bortezomib (Velcade®) (WO 02/059130 A1; WO 02/059131 A1) aus der Gruppe der Boronate und einer Verbindung gemäß der allgemeinen Formel (I) bereits in sehr niedrigen Konzentrationen die Replikation und damit die Infektionsausbreitung von HIV-1 hemmt (Ausführungsbeispiel 6). Hier zeigen jeweils unterschwellige Konzentrationen der beiden Substanzen bei gemeinsamer Applikation einen supraadditiven Effekt. Ein vergleichbarer Effekt konnte nach der kombinierten Applikation des spezifischen Immunoproteasominhibitors PR-957 (Synonym: ONX-0914; WO 2007/149512 A1; Muchamuel et al., 2009, Nat Med 15, 781-787) aus der Gruppe der Epoxyketone und einer erfindungsgemäßen Verbindung gemäß der allgemeinen Formel (I) bereits in sehr niedrigen Konzentrationen beobachtet werden (Ausführungsbeispiel 8). Auch hier zeigen jeweils unterschwellige Konzentrationen der beiden Substanzen bei gemeinsamer Applikation einen supraadditiven Effekt. Dies ist umso überraschender, als PR-619 als DUB-Inhibitor und Bortezomib oder PR-957 als Proteasom-Inhibitoren an unterschiedlichen Stellen des UPS ansetzen. Da der Wirkmechanismus aller Proteasom-Inhibitoren ähnlich ist, kann vernünftigerweise davon ausgegangen werden, dass auch die kombinierte Applikation einer Verbindung gemäß der allgemeinen Formel (I) mit einem anderen Proteasom-Inhibitor einen supraadditiven Effekt bei der Hemmung der Infektionsausbreitung von HIV-1 aufweist. Viele weisen zudem eine große strukturelle Ähnlichkeit entweder zu Bortezomib oder PR-619 auf.

Daher bezieht sich die vorliegende Anmeldung auch auf eine Kombination von mindestens einem Wirkstoff gemäß der allgemeinen Formel (I) und mindestens einem Proteasom-Inhibitor, bevorzugt auf eine Kombination von einem Wirkstoff gemäß der allgemeinen Formel (I) und Bortezomib oder PR-619 und besonders bevorzugt auf eine Kombination von PR-619 und Bortezomib sowie auf eine Kombination von PR-619 und PR-957, sowie die jeweiligen Salze und Hydrate der zuvor genannten Kombinationen zur Verwendung in der Behandlung und/oder Prophylaxe retroviraler Infektionen.

Weitere, nicht abschließende Beispiele für Proteasom-Inhibitoren sind Aldehyde wie MG-132, PSI, Fellutamide B; Boronate wie Delanzomib (CEP-18770), Ixazomib (MLN9708), MLN2238; MLN9074; Epoxyketone wie Epoximicin, Carfilzomib (PR-171), Oprozomib (ONX-0912; PR-047), YU-101; a-Ketoaldehyd-Proteasom-Inhibitoren; Beta-Lactone wie Omuralid, Salinosporamid A, PS-519, Marizomib (NPI-0052), Belactosin A, Belactosin C; Vinylsulfone wie ¹²¹I-NIP-L₃CS, MV151; Syrbactine wie SylA, GlbA; Lactame wie Lactacystin; Peptide wie PR-39; aus Bakterien isolierte Proteasom-Inhibitoren wie TMC-95A, Syringolin A, Glidobactin A, HT1171, GL5; sowie Chloroquin, 5-amino-8-hydroxychinolin (5AHQ), Clioquinol.

Hierdurch lassen sich synergistische Effekte in der Behandlung retroviraler Infektionen erzielen. Ein weiterer Vorteil ist, dass bei einer solchen Kombinationstherapie jeweils geringere Mengen des Einzelwirkstoffs eingesetzt werden können, was zu einer Verminderung der wirkstoffspezifischen Nebenwirkungen und gegebenenfalls zu einer Verringerung der bei HIV-Patienten bisher erheblichen Behandlungskosten führen kann. Gerade die schwerwiegenden Nebenwirkungen hatten den therapeutischen Einsatz von Proteasom-Inhibitoren bisher sehr limitiert. Ein weiterer Vorteil ist, dass bei einer Wirkung über mehr als einen Wirkmechanismus die Anzahl therapierefraktärer Patienten deutlich sinken wird. Des Weiteren wird durch eine derartige Kombinationstherapie, deren antiretrovirale Wirkung über verschiedene Wirkmechanismen vermittelt wird, das Risiko einer Resistenzbildung des Retrovirus, insbesondere von HIV, verringert.

Zu einer solchen Kombinationstherapie geeignete Reverse Transkriptase-Inhibitoren sind Nukleosidische Reverse Transkriptase-Inhibitoren (NRTI) und Nicht- Nukleosidische Reverse Transkriptase-Inhibitoren (NNRTI). Beispiele für NRTI sind, ohne abschließend zu sein, Abacavir, Didanosin, Emtricitabin, Lamivudin, Stavudin, Tenofovir, Zidovudin, Zalcitabin, Entecavir, Adefovir, Elvucitabin, Fosalvudin(-tidoxil), Fozivudintidoxil, Lagiciclovir, Alamifovir, Clevudin, Pradefovir, Telbivudin. Beispiele für NNRTI sind, ohne abschließend zu sein, Efavirenz, Etravirin, Nevirapin, Rilpivirin, Delavirdin, Emivirin, Lersivirin.

Zu der erfindungsgemäßen Kombinationstherapie geeignet sind Integrase-Inhibitoren wie beispielsweise Raltegravir, Elvitegravir, Dolutegravir, MK-2048.

Beispiele für zu der erfindungsgemäßen Kombinationstherapie geeignete HIV-Protease-Inhibitoren sind Saquinavir, Indinavir, Ritonavir, Nelfinavir, Amprenavir, Lopinavir, Atazanavir, Fosamprenavir, Tipranavir, Darunavir, Brecanavir, Mozenavir, Tipranavir.

Beispiele für zu der erfindungsgemäßen Kombinationstherapie geeignete Entry-Inhibitoren sind Enfuvirtid und Maraviroc.

Allgemeine Virostatika, die zu der erfindungsgemäßen Kombinationstherapie geeignet sind, können aus der Gruppe umfassend Ancriviroc, Aplaviroc, Cenicriviroc, Enfuvirtid, Maraviroc, Vicriviroc, Amantadin, Rimantadin, Pleconaril, Idoxuridin, Aciclovir, Brivudin, Famciclovir, Penciclovir, Sorivudin, Valaciclovir, Cidofovir, Ganciclovir, Valganciclovir, Sofosbusvir, Foscarnet, Ribavirin, Taribavirin, Filibuvir, Nesbuvir, Tegobuvir, Fosdevirin, Favipiravir, Merimepodib, Asunaprevir, Balapiravir, Boceprivir, Ciluprevir, Danoprevir, Daclatasvir, Narlaprevir, Telaprevir, Simeprevir, Vanipevir, Rupintrivir, Fomivirsen, Amenamevir, Alisporivir, Bevirimat, Letermovir, Laninamavir, Oseltamivir, Peramivir, Zanamivir ausgewählt werden.

Allgemeine Immunstimulanzien, die zu der erfindungsgemäßen Kombinationstherapie geeignet sind, können aus der Gruppe umfassend Interferone (α-, β-, γ-, τ-Interferon), Interleukine, CSF, PDGF, EGF, IGF, THF, Levamisol, Dimepranol, Inosin ausgewählt werden.

Des Weiteren sind erfindungsgemäß Kombinationen von PR-619 oder einem seiner Derivate mit Wirkstoffverstärkern wie Cobicistat möglich.

Besonders bevorzugt wird, wenn die erfindungsgemäße Substanz in der zuvor beschriebenen Wirkstoffkombination PR-619 ist.

Die Begriffe "Medizin" und "medizinisch" umfassen sowohl die Human- als auch die Veterinärmedizin.

Der Begriff "Organismus" bezieht sich auf ein Lebewesen, insbesondere Mensch oder Tier, das mit einem selbstregulierenden immunologischen System ausgestattet ist.

Der Begriff "Wirtsorganismus" wird im Sinne der Anmeldung für diejenigen Organismen verwendet, die von Viren, hier insbesondere Retroviren, nach einer Infektion mit ihnen zur Vermehrung genutzt werden.

Der Begriff "Wirkstoff" bezeichnet in dieser Anmeldung auf die erfindungsgemäß verwendeten Substanzen gemäß der allgemeinen Formel (I) und insbesondere auf PR-619. Zudem kann dieser Begriff weitere, bereits aus dem Stand der Technik bekannte pharmazeutische Wirkstoffe bezeichnen.

Die Begriffe "Zusammensetzung" und "pharmazeutische Zusammensetzung" umfassen mindestens einen Wirkstoff gemäß der allgemeinen Formel (I) in jeder pharmakologisch geeigneten, definierten Dosierung und Darreichungsform zusammen mit mindestens einem geeigneten Hilfsstoff, sowie alle Stoffe, die direkt oder indirekt als eine Kombination, Anhäufung, Komplexbildung oder Kristall aus den zuvor genannten Inhaltsstoffen entstehen, oder als Folge sonstiger Reaktionen oder Interaktionen, sowie optional mindestens einen weiteren, aus dem Stand der Technik bekannten pharmazeutischen Wirkstoff.

Der Ausdruck "Hilfsstoff" wird in dieser Anmeldung verwendet, um jeden Bestandteil einer pharmazeutischen Zusammensetzung neben dem Wirkstoff selbst zu beschreiben. Die Wahl eines geeigneten Hilfsstoffs hängt von Faktoren wie der Darreichungsform und der Dosierung ab sowie der Beeinflussung der Löslichkeit und Stabilität der Zusammensetzung durch den Hilfsstoff selbst.

Der Begriff "Wirkung" beschreibt die spezifische, dem jeweiligen Wirkstoff innewohnende Wirkweise im Rahmen der vorliegenden Erfindung.

Die Begriffe "Effekt", "therapeutischer Effekt", "Wirkung", "therapeutische Wirkung" in Hinblick auf mindestens einen Wirkstoff gemäß der allgemeinen Formel (I) beziehen sich auf die kausal eintretenden vorteilhaften Folgen an einem Organismus, dem der mindestens eine besagte Wirkstoff verabreicht worden ist.

"Therapeutisch wirksame Menge" meint im Sinne der Anmeldung, dass eine zur Erreichung des erwünschten therapeutischen Zweckes ausreichend große Menge des mindestens einen Wirkstoffs gemäß der allgemeinen Formel (I) einem Lebewesen oder einem Patienten, der eine derartige Behandlung benötigt, verabreicht wird.

Die Begriffe "gemeinsame Verabreichung", "kombinierte Verabreichung" oder "zeitgleiche Verabreichung" von mindestens einem pharmazeutischen Wirkstoff gemäß der allgemeinen Formel (I) und/oder mindestens einem pharmazeutischen Wirkstoff aus dem Stand der Technik umfasst die Verabreichung der besagten Wirkstoffe zum selben Zeitpunkt oder zu sachlich bedingten nahe beieinanderliegenden Zeitpunkten sowie zeitlich versetzte Verabreichungen der besagten Wirkstoffe innerhalb eines zusammenhängenden Experimentes. Die Reihenfolge der Verabreichung der besagten Wirkstoffe wird durch diese Begriffe nicht beschränkt. Der Fachmann wird keine Schwierigkeiten haben, sich aus seinem Fach- und Erfahrungswissen die beschriebenen Verabreichungen in ihrer zeitlichen und örtlichen Abfolge aus der Beschreibung zu erschließen.

Der Begriff "Lebewesen" bezieht sich auf jedes Tier, insbesondere Wirbeltier, inklusive den Menschen. Ein "Patient" im Sinne der Anmeldung ist ein Lebewesen, das an einer definierbaren und diagnostizierbaren Krankheit leidet und dem ein geeigneter Wirkstoff appliziert werden kann.

Die Begriffe "Prophylaxe", "Behandlung" und "Therapie" umfassen die Verabreichung mindestens eines Wirkstoffs gemäß der allgemeinen Formel (I), alleine oder in Kombination mit mindestens einem weiteren bereits bekannten pharmazeutischen Wirkstoff an ein Lebewesen, um die Ausbildung einer bestimmten Krankheit zu verhindern, zu inhibieren, die Symptome zu lindern oder einen Heilungsprozess der betreffenden Erkrankung zu initiieren.

Die Verbindungen der Formel (I) können als pharmazeutisch verträgliche Salze organischer und anorganischer Säuren vorliegen. Beispiele für geeignete Säuren sind Salzsäure, Bromwasserstoffsäure, Schwefelsäure, Phosphorsäure, Essigsäure, Zitronensäure, Oxalsäure, Malonsäure, Salizylsäure, p-Aminosalizylsäure, Äpfelsäure, Fumarsäure, Bernsteinsäure, Ascorbinsäure, Maleinsäure, Sulfonsäure, Phosphonsäure, Perchlorsäure, Salpetersäure, Ameisensäure, Propionsäure, Gluconsäure, Digluconsäure, Milchsäure, Weinsäure, Hydroxymaleinsäure, Brenztraubensäure, Phenylessigsäure, Benzoesäure, p-Aminobenzoesäure, p-Hydroxybenzoesäure, Dinitrobenzoesäure, Chlorbenzoesäure, Methansulfonsäure, Ethansulfonsäure, salpetrige Säure, Hydroxyethansulphonsäure, Ethylensulfonsäure, p-Toluolsulfonsäure, Naphthylsulfonsäure, Sulfanilsäure, Camphersulfonsäure, Alginsäure, Caprylsäure, Hippursäure, Pektinsäure, Phthalsäure, Chinasäure, Mandelsäure, o-Methylmandelsäure, Hyrogenbenzolsulfonsäure, Pikrinsäure, Adipinsäure, Cyclopentanpropionsäure, D-o-Toluylweinsäure, Tartronsäure, Benzolsulfonsäure, α-Toluylsäure, (o, m, p)-Toluylsäure, Naphthylaminsulfonsäure sowie weitere mineralische Säuren oder Carbonsäuren, die dem Fachmann wohl bekannt sind. Die Salze entstehen, indem die freie Base mit einer ausreichenden Menge der jeweiligen Säure in Kontakt gebracht wird, um das Salz auf herkömmliche Weise herzustellen. Pharmazeutisch verträgliche Salze sind im vorliegenden Zusammenhang als ein Wirkstoff zu verstehen, der eine Verbindung der Formel (I) in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch verträgliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in Bezug auf seine therapeutische Wirksamkeit im Körper positiv beeinflussen.

Die Verbindungen der Formel (I) können auch als Hydrate oder Solvate vorliegen. Als Solvate werden im Rahmen der Erfindung solche Formen der erfindungsgemäßen Verbindungen bezeichnet, welche durch Koordination mit Lösungsmittelmolekülen einen Komplex bilden. Hydrate sind eine spezielle Form der Solvate, bei denen die Koordination mit Wasser erfolgt.

Die Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen. Da sich die pharmazeutische Wirksamkeit der Racemate bzw. der Stereoisomeren der Verbindungen der Formel (I) unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle eines Racemats werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignete N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylat-Polymere). Als Laufmittel eignen sich hierfür wässrige oder alkoholische Lösungsmittelgemische wie z.B. Hexan/Isopropanol/Acetonitril z.B. im Verhältnis 82:15:3.

Pharmazeutische Formulierungen der Verbindungen der Formel (I) lassen sich zur Verabreichung über einen beliebigen geeigneten Weg, beispielsweise auf oralem (einschließlich bukkalem bzw. sublingualem), rektalem, vaginalem, nasalem, topischem (einschließlich bukkalem, sublingualem, konjunktivalem oder transdermalem), vaginalem oder parenteralem (einschließlich subkutanem, intramuskulärem, intravenösem. intraarteriellem oder intradermalem) Wege, applizieren.

Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

Die Verbindungen der Formel (I) können mit allen herkömmlich bekannten Trägerstoffen gemischt werden, bei festen Darreichungsformen z.B. pflanzliche und tierische Fette, Wachse, Paraffine, Stärke, Tragacanth, Cellulose-Derivaten, Polyethylenglykolen, Silikonen, Bentoniten, Kieselsäure, Talkum, Zinkoxid oder Gemische der vorgenannten Substanzen. Bei flüssigen Darreichungsformen und Emulsionen sind als Trägerstoffe z.B. Lösungsmittel, Solubulisierungsmittel, Emulgatoren wie z.B. Wasser, Ethanol, Isopropanol, Ethylcarbonat, Ethylacetat, Benzylalkohol, Benzylbenzoat, Propylenglykol, 1,3-Butylglykol, Baumwollsaatöl, Erdnussöl, Olivenöl, Rizinusöl, Sesamöl, Glycerol-Fettsäureester, Polyethylglykole, Fettsäureester von Sorbitan oder Gemische der vorgenannten Substanzen geeignet. Erfindungsgemäße Suspensionen können herkömmliche bekannte Trägerstoffe wie Verdünnungsmittel (z.B. Wasser, Ethanol oder Propylenglykol), ethoxylierte Isostearylalkohole, Polyoxyethylen und Polyoxyethylensorbitanester, mikrokristalline Zellulose, Bentonite, Agar-Agar, Tragacanth oder Gemische der vorgenannten Substanzen enthalten.

An die orale Verabreichung angepasste pharmazeutische Formulierungen können als separate Einheiten, wie z.B. Kapseln, Dragees, Pillen oder Tabletten; Puder, Pulver oder Granulate; Säfte, Sirups, Tropfen, Tees, Lösungen oder Suspensionen in wässrigen oder nicht-wässrigen Flüssigkeiten; essbare Schäume oder Schaumspeisen; oder Öl-in-Wasser-Flüssigemulsionen oder Wasser-in-ÖI-Flüssigemulsionen dargereicht werden.

So lässt sich beispielsweise bei der oralen Verabreichung in Form einer Tablette oder Kapsel die Wirkstoffkomponente mit einem oralen, nicht-toxischen und pharmazeutisch verträglichen inerten Trägerstoff, wie z.B. Ethanol, Glycerin, Wasser u.a. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem essbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein. Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Calciumstearat oder Polyethylenglykol in Festform können dem Pulvergemisch vor dem Füllvorgang zugesetzt werden. Ein Sprengmittel oder Lösungsvermittler, wie z.B. Agar-Agar, Calciumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- Gleit- und Sprengmittel sowie Farbstoffe ebenfalls in das Gemisch eingearbeitet werden.

Als Bindemittel werden Substanzen bezeichnet, die Pulver binden oder miteinander verkleben und sie so durch Granulabildung kohäsiv werden lassen. Sie dienen als "Klebstoff" der Formulierung. Bindemittel erhöhen die Kohäsionsstärke der vorliegenden Verdünnungs- oder Stellmittel.

Zu den geeigneten Bindemitteln gehören Stärke aus Weizen, Mais, Reis oder Kartoffel, Gelatine, natürliche Zucker, wie z.B. Glucose, Sucrose oder Beta-Lactose, Süßstoffe aus Mais, natürliche und synthetische Gummi, wie z.B. Akazia, Tragant oder Ammoniumcalciumalginat, Natriumalginat, Carboxymethylzellulose, Natriumcarboxymethylzellulose, Hydroxypropylcarboxymethylzellulose, Polyethylenglykol, Polyvinylpyrrolidon, Magnesiumaluminiumsilicat, Wachse, u.a. Der Anteil des Bindemittels in dem Gemisch kann 1 - 30 Gewichts-%, bevorzugt 2 - 20 Gewichts-%, weiter bevorzugt 3 - 10 Gewichts-% und am meisten bevorzugt 3 - 6 Gewichts-% betragen.

Der Begriff Schmiermittel bezieht sich auf Substanzen, die der Dosierungsform beigefügt werden, um Tabletten, Granulate etc. nach ihrer Verpressung leichter aus der Pressform oder der Austrittsdüse lösen zu können, indem sie die Reibung oder den Abrieb verringern. Schmiermittel werden gewöhnlich kurz vor der Verpressung zugegeben, da sie auf der Oberfläche der Granula sowie zwischen ihnen und den Bauteilen der Tablettenpresse vorhanden sein sollen. Die Menge des Schmiermittels kann zwischen 0,05 und 15 Gewichts-% der Zusammensetzung variieren, bevorzugt zwischen 0,2 und 5 Gewichts-%, weiter bevorzugt zwischen 0,3 und 3 Gewichts-%, und am meisten bevorzugt zwischen 0,3 und 1,5 Gewichts-%.

Zu den in diesen Darreichungsformen verwendeten Schmiermitteln gehören Natriumoleat, Metallstearate wie Natriumstearat, Calciumstearat, Kaliumstearat und Magnesiumstearat, Stearinsäure, Natriumbenzoat, Natriumacetat, Natriumchlorid, Borsäure, Wachse mit einem hohen Schmelzpunkt, Polyethylenglykole u.a.

Gleitmittel sind Materialien, die ein Zusammenbacken unterbinden und die Fließeigenschaften der Granulationen verbessern, so dass der Fluss glatt und gleichmäßig erfolgt.

Geeignete Gleitmittel umfassen Silikondioxid, Magnesiumstearat, Natriumstearat und Talkum. Die Menge des Gleitmittels in der Zusammensetzung kann zwischen 0,01 und 10 Gewichts-% variieren, bevorzugt zwischen 0,1 und 7 Gewichts-%, weiter bevorzugt zwischen 0,2 und 5 Gewichts-% und am meisten bevorzugt zwischen 0,5 und 2 Gewichts-%.

Der Begriff Sprengmittel beziehen sich auf Materialien, die der Zusammensetzung zugegeben werden, damit dieses leichter auseinandergebrochen werden können.

Zu den Sprengmitteln gehören, ohne darauf beschränkt zu sein, Stärke, in kaltem Wasser lösliche Stärken wie Carboxymethylstärke, Zellulosederivate wie Methylzellulose und Natriumcarboxymethylcellulose, mikrokristalline Zellulosen und kreuzvernetzte mikrokristalline Zellulosen wie Croscarmellose-Natrium, natürliche und synthetische Gummi wie Guar, Agar, Karaya, Johannisbrotkernmehl, Tragant, Tonerden wie Bentonit, Xanthangummi, Alginate wie Alginsäure und Natriumalginat, aufschäumende Gemische u.a. Die Quellung unterstützen beispielsweise Stärke, Cellulose-Derivate, Alginate, Polysaccharide, Dextrane, quervernetztes Polyvinylpyrrolidon. Die Menge der Sprengmittel in der Zusammensetzung kann zwischen 1 und 40 Gewichts-% variieren, bevorzugter zwischen 3 und 20 Gewichts-% und am meisten bevorzugt zwischen 5 und 10 Gewichts-%.

Farbstoffe sind Hilfsmittel, die der Zusammensetzung bzw. der Darreichungsform eine Färbung verleihen. Diese Hilfsmittel können Lebensmittelfarbstoffe sein. Diese können auch auf einem geeigneten Adsorptionsmittel wie Tonerde oder Aluminiumoxid adsorbiert sein. Die Menge der Farbstoffe kann zwischen 0,01 und 10 Gewichts-% der Zusammensetzung variieren, bevorzugt zwischen 0,05 und 6 Gewichts-%, weiter bevorzugt zwischen 0,1 und 4 Gewichts-% und am meisten bevorzugt zwischen 0,1 und 1 Gewichts-%.

Die Tabletten werden formuliert, indem beispielsweise ein Pulvergemisch hergestellt, granuliert oder trockenverpresst wird, ein Schmiermittel und ein Sprengmittel zugegeben werden und das Ganze zu Tabletten verpresst wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dicalciumphosphat, vermischt wird. Das Pulvergemisch lässt sich granulieren, indem es mit einem Bindemittel, wie z.B. Sirup, Stärkepaste, Pflanzenschleim (z.B. Acacia) oder Lösungen aus Zellulose- oder Polymer- materialen benetzt und durch ein Sieb gepresst wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengussformen zu verhindern. Das gefettete Gemisch wird dann zu Tabletten verpresst. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpresst werden.

Flüssige Darreichungsformen umfassen Lösungen, Suspensionen und Emulsionen. Beispiele sind Wasser und Wasser-Propylen-Glykol-Lösungen für parenterale Injektionen oder der Zusatz von Süßmitteln und Trübungsmitteln für orale Lösungen, Suspensionen und Emulsionen. Flüssige Darreichungsformen können auch Lösungen für intranasale Verabreichung umfassen.

Des Weiteren können Pufferlösungen Bestandteile pharmazeutischer Zusammensetzungen sein. Die Begriffe Puffer, Puffersystem und Pufferlösung beziehen sich auf die Fähigkeit eines Systems, insbesondere einer wässrigen Lösung, einer pH-Veränderung durch Zugabe von Säure oder Base oder durch Verdünnung mit einem Lösungsmittel zu widerstehen.

Puffersysteme können aus der Gruppe bestehend aus Format, Lactat, Benzoesäure, Oxalat, Fumarat, Anilin, Acetat-Puffer, Zitrat-Puffer, Glutamat-Puffer, Phosphat-Puffer, Succinat, Pyridin, Phthalat, Histidin, MES (2-(N-Morpholino)ethansulfonsäure), Maleinsäure, Cacodylat (Dimethylarsenat), Carbonsäure, ADA (N-(2-Acetamido)iminodiessigsäure), PIPES (4-piperazin-bis-ethansulfonsäure); BIS-TRIS-Propan (1,3-Bis[tris(hydroxymethyl)methylamino]-propan), Ethylendiamin, ACES (2-[(2-Amino-2-oxoethyl)amino]ethansulfosäure), Imidazol, MOPS (3-(N-Morphin)-propansulfonsäure), Diethylmalonsäure, TES (2-[Tris(hydroxymethyl)-methyl]aminoethansulfonsäure) und HEPES (N-2-Hydroxylethylpiperazin-N'-2-ethansulfonsäure-Puffer) sowie weitere Puffer mit einem pKₐ zwischen 3,8 bis 7,7 ausgewählt werden.

Bevorzugt werden Carbonsäure-Puffer wie Acetat- und Dicarbonsäure-Puffer wie Fumarat, Tartrat und Phthalat sowie Tricarbonsäure-Puffer wie Citrat. Eine weitere Gruppe bevorzugter Puffer sind anorganische Puffer wie Sulfat-, Borat-, Carbonat-, Oxalat-, Calciumhydroxid- und Phosphat-Puffer. Noch eine weitere Gruppe bevorzugter Puffer sind Stickstoff-haltige Puffer wie Imidazol, Diethylendiamin und Piperazin. Des Weiteren werden Sulfonsäure-Puffer wie TES, HEPES, ACES, PIPES, [(2-Hydroxy-1,1-bis(hydroxymethyl)ethyl)amino]-1-propansulfonsäure (TAPS), 4-(2-Hydroxyethyl)piperazin-1-propansulfonsäure (EPPS), 4-Morpholinepropansulfonsäure (MOPS) und N,N-Bis(2-hydroxyethyl)-2-aminoethansulfonsäure (BES) bevorzugt. Eine weitere Gruppe bevorzugter Puffer sind Glycin, Glycylglycin, Glycylglycylglycin, N,N-Bis(2-hydroxyethyl)glycin und N-[2-Hydroxy-1,1-bis(hydroxy-methyl)ethyl]glycin (Tricin). Bevorzugt werden auch 23

Aminosäurepuffer wie Glycin, Alanin, Valin, Leucin, Isoleucin, Serin, Threonin, Phenylalanin, Tyrosin, Tryptophan, Lysin, Arginin, Histidin, Aspartat, Glutamat, Asparagin, Glutamin, Cystein, Methionin, Prolin, 4-Hydroxyprolin, N,N,N-Trimethyllysin, 3-Methylhistidin, 5-Hydroxylysin, o-Phosphoserin, Gamma]-carboxyglutamat, [Epsilon]-N-Acetyllysin, [Omega]-N-Methylarginin, Citrullin, Ornithin und deren Derivate.

Konservierungsmittel für flüssige Darreichungsformen können bei Bedarf eingesetzt und dabei ausgewählt werden aus Kaliumsorbat, Methyl-Ethylparaben, Natriumbenzoat und ähnlichen dem Fachmann zu diesem Zweck bekannten Substanzen oder Gemischen hiervon.

Eine besonders bevorzugte pharmazeutische Zusammensetzung ist ein Lyophilisat (eine gefriergetrocknete Zubereitung), das zur Verabreichung per Inhalation oder intravenöser Injektion geeignet ist. Zu deren Herstellung wird eine erfindungsgemäße Verbindung in einer 4 - 5% Mannitol-Lösung solubilisiert und diese Lösung dann lyophilisiert. Die Mannitol-Lösung kann auch in einer geeigneten Puffer-Lösung wie zuvor beschrieben zubereitet werden. Weitere Beispiele geeigneter Kryo-/Lyoprotektantien (auch als Stellmittel oder Stabilisatoren) sind Thiol-freies Albumin, Immunglobulin, Polyalkylenoxid (d.h. PEG, Polypropylenglykole), Trehalose, Glucose, Sucrose, Sorbitol, Dextran, Maltose, Raffinose, Stachyose und andere Saccharide. Mannitol wird bevorzugt. Sie können in üblichen Mengen bei den dem Fachmann bekannten Lyophilisierungsverfahren eingesetzt werden.

Zur Herstellung einer Darreichungsform als Zäpfchen mit den Verbindungen der Formel (I) werden Wachse mit einem niedrigen Schmelzpunkt sowie ein Gemisch aus Fettsäureglyceriden wie Kakaobutter zuerst zum Schmelzen gebracht, dann der Wirkstoff durch Rühren oder andere Mischmethoden homogen darin dispergiert. Die geschmolzene homogene Mischung wird dann in geeignete Gussformen überführt, abkühlen gelassen und somit verfestigt.

Für topische Applikationen der Verbindungen der Formel (I) sind Cremes, Emulsionen, Lotionen, Gele, Pasten, Salben und Suspensionen geeignet.

Als oberflächenaktive Solubilisatoren (Lösungsvermittler) eignen sich beispielsweise Diethylenglykolmonoethylether, Polyethylpropylenglykol-Copolymere, Cyclodextrine, Glycerylmonostearate wie z.B. Solutol HS 15 (Macrogol-15-hydroxystearat von BASF, PEG-660-15-hydroxystearate), Sorbitanester, Polyoxyethylensorbitansäureester, Polyvinylalkohol, Natriumlaurylsulfat, (anionische) Glycerylmonooleate, etc.

Als Emulgatoren kommen beispielsweise die folgenden anionischen und nichtionischen Emulgatoren in Frage: anionische Emulgatorwachse, Cetylalkohol, Cetylstearylalkohol, Stearinsäure, Ölsäure, Polyoxyethylen-Polyoxypropylen-Blockpolymere, Anlagerungsprodukte von 2 bis 60 Mol Ethylenoxid an Rizinusöl und/oder gehärtetes Ricinusöl, Wollwachsöl (Lanolin), Sorbitanester, Polyoxyethylenalkyl-ester, PolyoxyethylenSorbitanfettsäureester oder Polyvinylalkohol. Bevorzugt sind Glycerinmonooleat, Stearinsäure und Phospholipide wie z.B. Lezithin.

Als Triglyceride kommen mittelkettige und hochmolekulare Triglyceride in Frage. Mittelkettige Triglyceride sind Glycerinester der Fettsäuren mit nur 6-12 Kohlenstoffatomen, wie z.B. Capryl-Caprinsäuretriglycerid. Hochmolekulare Triglyceride sind Glycerinfettsäureester mit langkettigen Fettsäuren. Sie sind z.B. aus verschiedenen natürlichen Fetten hergestellte Triglyceridgemische. Bevorzugt werden mittelkettige Triglyceride eingesetzt, insbesondere Capryl-Caprinsäuretriglycerid.

Zu den geeigneten Eindringungsverstärker (penetration enhancers) zählen z.B. Isopropylmyristat, Ölsäure, Natriumlaurylsulfat und 1,2-Propandiol. Bevorzugt ist 1,2-Propandiol.

Typische Beispiele für Konservierungsmittel, die für eine topische Applikation geeignet sind, sind Benzylbenzoate, Benzoesaure, Benzylalkohol, Benzalkoniumchlorid, N-Cetyl-N,N,-Trimethylammoniumbromid (Cetrimid, Fa. Merck), Chlorhexidin, Chlorbutanol, Chlorcresol, Imidurea, die Parabene, wie Methyl-, Ethyl-, Propyl- oder Butylparaben, Natriummethylparaben, Natriumpropylparaben, Kaliumsorbat, Natrium- benzoat, Natriumpropionat, Phenol, Phenoxyethanol, Phenylethylalkohol, Phenylmercuriacetat, Phenylmercuriborat, Phenylmercurinitrate, Sorbinsäure oder Thiomersal (Natriumethylmercurithiosalicylat). Bevorzugt sind Methylparaben, Propylparaben sowie Natriummethylparaben und Natriumpropylparaben.

Bei einigen topischen Applikationen ist es vorteilhaft, Antioxidantien zuzusetzen. Geeignete Beispiele hierfür sind Natriummetabisulfit, alpha-Tocopherol, Ascorbinsäure, Maleinsäure, Natriumascorbat, Ascorbylpalmitat, butyliertes Hydroxyanisol, butyliertes Hydroxytoluol, Fumarsäure oder Propylgallat. Bevorzugtes Antioxidantium ist Natriummetabisulfit.

Als pH-regulierendes Mittel bei topischen Darreichungsformen kommen z.B. Natriumhydroxid, Salzsäure, Puffersubstanzen, wie z.B. Natriumdihydrogenphosphat oder Dinatriumhydrogenphosphat in Frage.

Creme-Zubereitungen können ferner weitere Hilfs- und Zusatzstoffe enthalten, wie z.B. Fettungsmittel, Lösungsmittel, Konsistenzgeber oder Hydrotrope, zur Verbesserung des Fließverhaltens. Dabei können von den vorgehend angegebenen Additiven bzw. Zusatzstoffen jeweils einzelne oder auch mehrere Stoffe derselben Gruppe im Gemisch anwesend sein.

Als Fettungsmittel eignen sich z.B. Ölsäuredecylester, hydriertes Ricinusöl, leichtes Mineralöl, Mineralöl, Polyethylenglykol, Natriumlaurylsulfat.

Als Lösungsmittel kommen Maiskeimöl, Baumwollsaatöl, Erdnussöl, Sesamöl, Sojabohnenöl, Ethyloleat, Glycerin, Isopropylmyristat, Isopropylpalmitat, Polyethylenglykol oder Polypropylenglykol in Frage.

Als Konsistenzgeber eignen sich beispielsweise Cetylalkohol, Cetylesterwachs, hydriertes Ricinusöl, mikrokristalline Wachse, nichtionische Emulgatorwachse, Bienenwachs, Paraffin oder Stearylalkohol.

Geeignete Hydrotope sind Alkohole, wie beispielsweise Ethanol, Isopropylalkohol oder Polyole, wie z.B. Glycerin.

Die Zubereitungen weisen weiterhin Zusatzstoffe auf. Diese werden vorzugsweise ausgewählt aus Aromen/Geschmacksstoffen, insbesondere ätherischen Ölen, Vitaminen, sowie galenischen Hilfsstoffen, ausgewählt aus Zuckern,

Zuckeraustauschstoffen, Zuckerersatzstoffen, Säuerungsmitteln, Lösungsvermittlern wie z. B. Wasser, Glykol, Glycerin, Verdickern, Süßungsmitteln, Farbstoffen oder Konservierungsmitteln oder Kombinationen hiervon, auch in Abhängigkeit von der galenischen Darreichungsform.

Zu den geeigneten Aromen und Geschmacksstoffen gehören vor allem ätherische Öle, die als Aroma- bzw. als Geschmacksstoffe verwendbar sind. Dabei handelt es sich um im Allgemeinen flüchtige Extrakte aus Pflanzen, Pflanzenteilen mit dem dafür charakteristischen Geruch, die vor allem durch Wasserdampfdestillation aus Pflanzen oder Pflanzenteilen gewonnen werden können.

Beispielhaft können hier genannt werden: ätherische Öle bzw. Aromastoffe aus Salbei, Nelken, Kamille, Anis, Sternanis, Thymian, Teebaum, Pfefferminze, Minzöl, (Menthol, Cineol), Eukalyptusöl, Mango, Feigen, Lavendelöl, Kamillenblüten, Kiefernnadel, Zypresse, Orangen, Rosenholz, Pflaumen-, Johannisbeer-, Kirscharoma, Birkenblätter, Zimt, Limetten, Orangen, Grapefruit, Mandarine, Wacholder, Baldrian, Zitronenmelisse, Zitronengras, Palmarosa, Cranberry, Granatapfel, Rosmarin, Ingwer, Ananas, Guave, Echinacea, Efeublätterextrakt, Heidelbeere, Kaki, Melonen u. ä. oder Mischungen hiervon wie Mischungen aus Menthol, Pfefferminz- und Sternanisöl oder Menthol und Kirscharoma. Diese Aroma- bzw. Geschmacksstoffe können in Mengen von 0,0001 bis 10 Gewichts-%

(insbesondere als Mischung), vor allem 0,001 bis 6 Gewichts-%, bevorzugt 0,001 bis 4 Gewichts-%, besonders bevorzugt 0,01 bis 1 Gewichts-%, bezogen auf die Gesamtzubereitung vorliegen. Anwendungsbedingt oder einzelfallabhängig kann es von Vorteil sein, hiervon abweichende Mengen einzusetzen.

Somit eignen sich die Verbindungen der Formel (I) sowie die zuvor beschriebenen Wirkstoffkombinationen zur Verwendung zur Herstellung einer Formulierung zur oralen Verabreichung.

Gleichermaßen eignen sich die Verbindungen der Formel (I) sowie die zuvor beschriebenen Wirkstoffkombinationen zur Verwendung zur Herstellung einer Formulierung als Lyophilisat oder einer flüssigen Formulierung.

Offenbart ist ebenfalls die Verwendung der Verbindungen der Formel (I) sowie der zuvor beschriebenen Wirkstoffkombinationen zur Herstellung einer topischen Formulierung.

Die vorliegende Offenbarung bezieht sich auch auf eine pharmazeutische Zusammensetzung, die mindestens eine Verbindung der Formel (I) oder eine der zuvor beschriebenen Wirkstoffkombinationen zusammen mit mindestens einem pharmazeutisch verträglichen Trägerstoff, Hilfsstoff, Verdünnungsmittel, Kryoprotektivum und/oder Lyoprotektivum enthält. Bevorzugt wird, wenn die besagte pharmazeutische Zusammensetzung zur oralen, parenteralen, topischen Verabreichung und/oder zur Verabreichung per Inhalation geeignet ist.

Erfindungsgemäß ist die besagte pharmazeutische Zusammensetzung zur Behandlung und/oder Prophylaxe von retroviralen Infektionen geeignet. Besonders bevorzugt wird, wenn die besagte pharmazeutische Zusammensetzung zur Behandlung und/oder Prophylaxe von retroviralen Infektionen PR-619 als Wirkstoff enthält.

Die zuvor beschriebenen Formulierungen und pharmazeutischen Zusammensetzungen können neben den Verbindungen der Formel (I) auch mindestens einen weiteren Wirkstoff enthalten. Geeignet als Kombinationswirkstoff sind die oben genannten Wirkstoffe zur Kombinationstherapie. Bevorzugt wird, wenn die Substanz in der besagten Kombinationsformulierung oder Kombinationszusammensetzung PR-619 ist.

Offenbart wird ebenfalls ein Verfahren zur Behandlung und/oder Prophylaxe retroviraler Infektionen bei einem Mensch oder einem Wirbeltier, das die Verabreichung einer pharmazeutisch wirksamen Menge einer Verbindung gemäß der allgemeinen Formel (I) oder eines ihrer Salze, Solvate und/oder Hydrate beinhaltet, die geeignet ist, bei besagter retroviraler Infektion wirksam zu sein.

Bevorzugt wird, wenn die retrovirale Infektion aus dem zuvor beschriebenen Behandlungsverfahren HIV-1-Infektion, HIV-2-Infektion, AIDS, FIV-Infektion und SIV-Infektion ist.

Ebenfalls bevorzugt wird, wenn in dem zuvor beschriebenen Behandlungsverfahren der mindestens eine erfindungsgemäße Wirkstoff zusätzlich mit einer pharmazeutisch wirksamen Menge mindestens eines weiteren Wirkstoffes verabreicht wird, wobei der besagte weitere Wirkstoff aus einer Gruppe umfassend Proteasom-Inhibitoren, Reverse Transkriptase-Inhibitoren, Integrase-Inhibitoren, Protease-Inhibitoren, Entry-Inhibitoren, HIV-Vakzine, Virostatika und Immunstimulanzien ausgewählt wird und die Wirkstoffkombination geeignet ist, bei besagter retroviraler Infektion wirksam zu sein.

Bei diesem kombinierten Behandlungsverfahren kann die Wirkstoffkombination entweder getrennt oder in einer gemeinsamen Formulierung oder Zusammensetzung verabreicht werden.

Besonders bevorzugt wird, wenn die Verbindung der Formel (I) in den zuvor beschriebenen Behandlungsverfahren PR-619 ist.

Zudem wird bevorzugt, wenn die Wirkstoffkombination in den zuvor beschriebenen Behandlungsverfahren PR-619 und Bortezomib oder PR-957 umfasst.

### Beispiele

### Beispiel 1: Hemmung der Prozessierung der HIV-1 Gag Strukturproteine

Um zu untersuchen, ob PR-619 und seine Derivate die Prozessierung der HIV-1 Gag Strukturproteine hemmen, wurden Western Blot (WB)- Analysen durchgeführt. Hierfür wurden Kulturen von HeLa-Zellen mit dem pNLΔenv-Plasmid transfiziert, welches für alle HIV-1 Proteine kodiert, ausgenommen die Env-Hüllproteine. Dadurch wird die Produktion von *virus-like particles* (VLPs) ermöglicht, was ein etabliertes *in vitro* Modell für die Erforschung von HIV-1 ist. 24h nach der Transfektion wurden die Zellen in Reaktionsgefäße überführt und es erfolgte eine Vorinkubation für 1h mit PR-619. Anschließend wurden die Zellen dreimal mit PBS gewaschen und für 4 h in frischem RPMI-Medium entweder mit 20 µM PR-619 (Merck-Millipore) oder der entsprechenden Menge DMSO als Lösungsmittelkontrolle bei 37°C inkubiert.

Daraufhin erfolgte mittels Zentrifugation die Auftrennung in Zell- und VLP-Fraktion. Die VLPs wurden aus den Zellkulturüberständen durch Zentrifugation über ein 20% Sucrose-Kissen gereinigt. Die Zellen wurden anschließend mit PBS gewaschen und mit RIPA-Puffer lysiert. Die Proteinkonzentrationen wurden mittels des Bradford Proteinassays bestimmt und für die jeweiligen Lysate aneinander angeglichen. Die zytosolische Fraktion der Zelllysate wurde in SDS-Sample-Puffer denaturiert, mittels SDS-Gelelektophorese separiert und auf eine Nitrozellulosemembran transferiert. Die Gag-Proteine wurden mit einem Gag-spezifischen Antikörper (AK) und einem Meerrettichperoxidase-gekoppelten Sekundärreagenz mit Hilfe einer Elektrochemolumineszenz-Reaktion sichtbar gemacht.

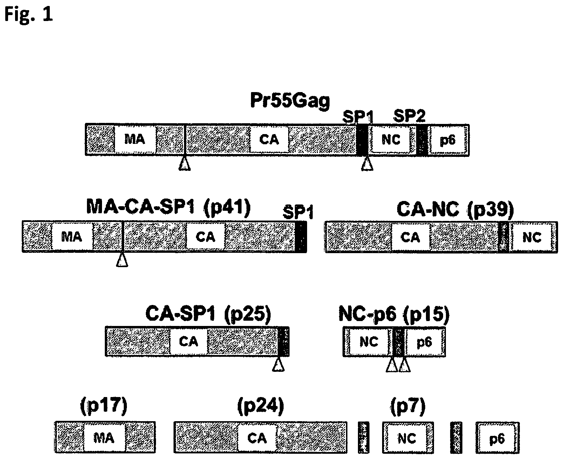

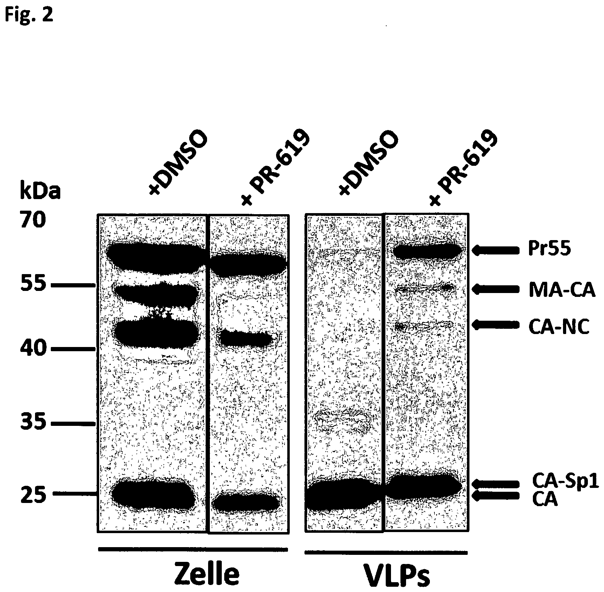

Die Ergebnisse des WB zeigen, dass PR-619 die Gag-Prozessierung wesentlich hemmt. Der beobachtete Defekt in der Prozessierung beeinflusst mehrere Schritte der Reifung des Gag-Polyproteins Pr55. Die vollständige Spaltung von HIV-1 Pr55 führt allgemein zu der Entstehung der reifen Gag-Proteine MA, CA, NC und p6Gag, sowie zwei kleinerer Spacer-Peptide (SP2 und SP1), welche die einzelnen Domänen miteinander verbinden (Fig. 1). Mehrere dieser Spaltprozesse sind im Prozess der Gag-Prozessierung durch die Einwirkung von PR-619 gehemmt, da das Auftreten von Intermediaten der Gag-Prozessierung wie z.B. MA-CA (p41), p39 (CA-NC) oder CA - dem 14 Aminosäuren langen SP1 - (p25CA) nach Blockade der DUB-Aktivität beobachtet wurde (Fig. 2).

Eine densitometrische Auswertung der Gag-Prozessierung in der VLP-Fraktion erfolgte mit dem Analyseprogramm AIDA®. Die densitometrische Auswertung ermöglicht die Quantifizierung von Signalintensitäten im Western Blot und somit Rückschlüsse auf die Quantität eines bestimmten Proteins in der Probe. Dabei wurde die Intensitätsstärke von CA ins Verhältnis gesetzt zur Summe der Intensitätsstärke vom Gesamtgehalt an Gag, und dadurch der prozentuale Anteil von CA in den VLPs angegeben.

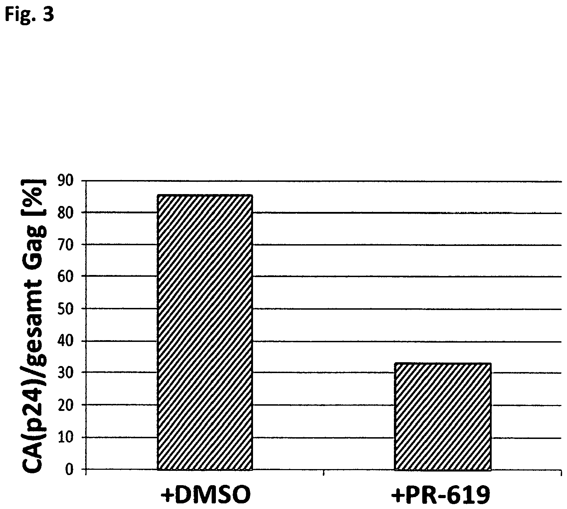

Die Auswertung zeigt deutlich, dass nach Zugabe von PR-619 die Entstehung des Gag-Hauptprozessierungsproduktes CA, welches ein Merkmal infektiöser HIV-1 Virionen ist, deutlich gehemmt wird (Fig. 3).

Zusammengefasst wird festgestellt, dass PR-619 mit der proteolytischen Reifung von Gag-Proteinen interferiert und die Entstehung des Gag-Hauptprozessierungsproduktes CA gehemmt wird.

### Beispiel 2: Der DUB-Inhibitor PR-619 hemmt konzentrationsabhängig die HIV-1-Replikation in primären T-Zellen und Makrophagen, ohne die Vitalität der Wirtszelle zu beeinflussen.

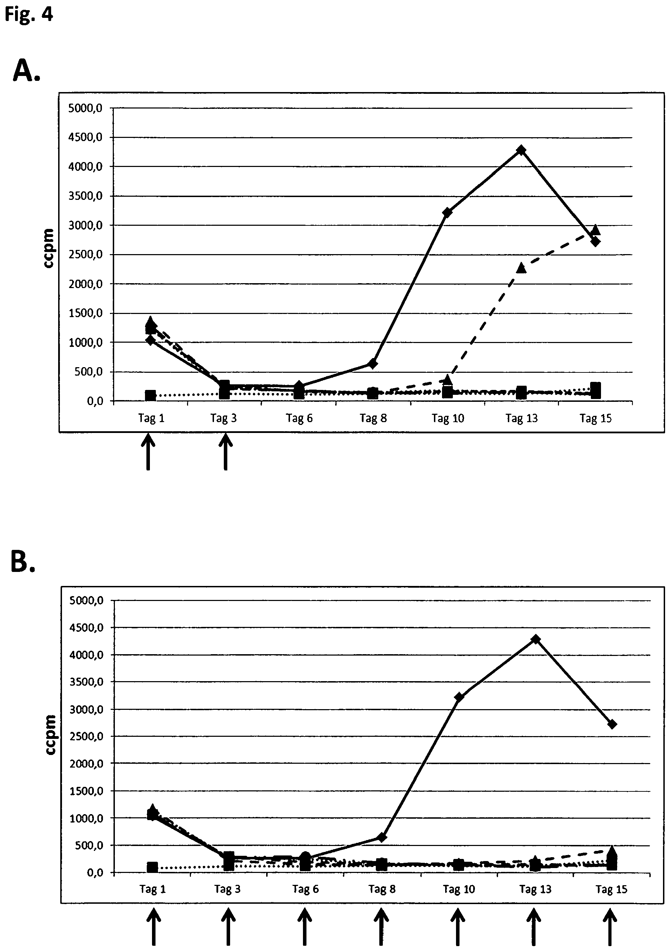

Nachdem im vorhergehenden Beispiel gezeigt werden konnte, dass PR-619 die späten Prozesse der Gag-Prozessierung hemmt, wurde dessen Effekt auf die Infektionsausbreitung untersucht. Erfindungsgemäß wurden dazu isolierte primäre humane Peripheral Blood Mononuclear Cell (PBMC)-Kulturen mit dem Virusstamm HIV-1_{NL4-3}X4-troph bzw. HIV-1_{NL4-3} R5-troph infiziert. Einen Tag nach der Infektion wurden die Zellen in PBS (phosphate buffer saline) gewaschen, mit frischem Medium versetzt, welches den DUB-Inhibitor PR-619 in verschiedenen nicht zytotoxischen Konzentrationen (3,5 µM, 7 µM, 14 µM, 28 µM) enthält. Die Behandlung mit DUB-Inhibitoren erfolgte entweder über den gesamten Zeitraum ("permanente Behandlung") oder nur an Tag 1 und Tag 3 nach der Infektion ("strukturierte Behandlung"). Bei der Infektion mit HIV-1_{NL4-3} R5-troph erfolgte nur eine permanente Behandlung. Alle 2-3 Tage wurden Proben von Zellkultur-Überständen abgenommen, eingefroren und später zur Bestimmung der Reverse Transkriptase -Aktivität eingesetzt. Gleichzeitig wurden 80% des Zellkultur-Mediums erneuert und mit frischem PR-619 versetzt. Die Reverse Transkriptase-Aktivität (ccpm) wurde in den zellfreien Zellkultur-Überständen bestimmt und gegen die Zeit aufgetragen (Fig. 4A und 4B; schwarze Pfeile zeigen die Behandlung mit PR-619 an).

Dabei zeigt sich in T-Zellen eine dosisabhängige Inhibierung der HIV-1-Replikation. Bereits ab einer Konzentration von 3,5 µM bewirkt der DUB-Inhibitor PR-619 eine deutliche (bei strukturierter Behandlung) bzw. vollständige (bei permanenter Behandlung) Hemmung der HIV-1-Replikation (Fig. 4A und 4B). Interessanterweise beginnt HIV-1 eine Woche nach Beendigung der strukturierten Behandlung mit 3,5 µM PR-619 wieder zu replizieren (Fig. 4A). Dies zeigt, dass die beobachteten antiretroviralen Effekte nicht auf eine unspezifische zytotoxische Wirkung von PR-619 beruhen. Diese Beobachtung unterstreicht den spezifischen Effekt der DUB-Inhibitoren auf die HIV-1-Replikation.

Bei sechs verschiedenen Spendern wurde mittels der Replikationsprofile von HIV-1 nach Infektion von PBMCs aus mit T-Zell-trophem Virus die *area under the curve* (AUC) bestimmt, welche die Replikationskapazität von HIV-1 repräsentiert.

Die Auswertung von 6 Replikationsprofilen zeigt sowohl bei der strukturierten als auch bei der permanenten Behandlung mit PR-619 eine signifikante, dosisabhängige Reduktion der HIV-1 Replikationskapazität in T-Zellen. Bei einer Konzentration von 3,5 µM PR-619 war bei der strukturierten Behandlung eine Reduktion der Replikationskapazität um 23% (+/- 14%), bei 7 µM um 94% (+/- 14%) und bei 14 µM um 100% zu beobachten. Bei der permanenten Behandlung erfolgte bei 3,5 µM eine Reduktion um 50 % (+/- 16%), bei 7 µM eine Reduktion um 97 % (+/- 15%) und bei 14 µM um 100 %.

Um zu überprüfen, ob die Replikation auch in Makrophagen, einem potentiellen Reservoir für HIV-1, nach Zugabe von PR-619 gehemmt wird, wurde nach Infektion mit Makrophagentrophem Virus, das Replikationsprofil aus 5 Spendern ausgewertet. Bei der permanenten Behandlung erfolgte bei 3,5 µM eine Reduktion um 35 % (+/- 8%), bei 7 µM eine Reduktion um 60 % (+/- 9 %) und bei 14 µM um 100 %.

Zusammengefasst lässt sich feststellen, dass die erfindungsgemäße Verwendung von PR-619 sowohl bei strukturierter, als auch bei permanenter Behandlung dosisabhängig zu einer vollständigen Hemmung der HIV-1-Replikation in T-Zellen und Makrophagen führt.

### Beispiel 3: Vergleich der Wirkung von PR-619 mit den DUB-Inhibitoren P005091, WP1130 und P22077

Vergleichsweise wurde der bei PR-619 gefundene antiretrovirale Effekt mit dem von drei weiteren DUB-Inhibitoren P005091, WP1130 und P22077 verglichen. Die Versuche wurden wie unter Beispiel 2 beschrieben durchgeführt.

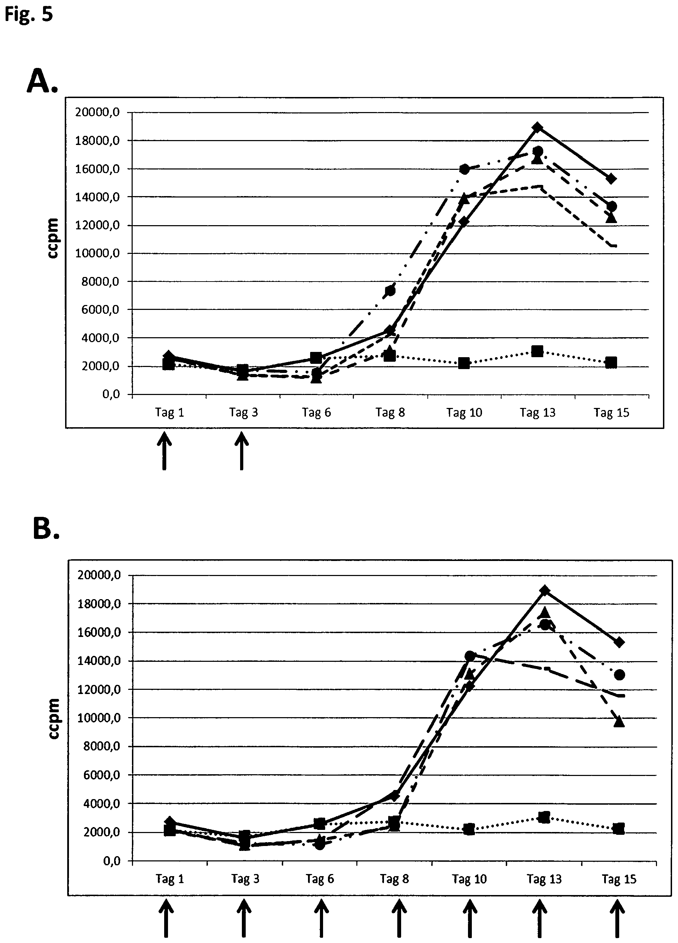

Dabei zeigte sich, dass der DUB-Inhibitor P005091 (Tocris), welcher sehr spezifisch USP7 hemmt, in den verwendeten, nicht zytotoxischen Konzentrationen von 0,75 µM, 1,5 µM und 3 µM die HIV-1-Replikation sowohl in T-Zellen als auch in Makrophagen nicht hemmt (Fig. 5A und 5B). Die statistische Auswertung von 5 Replikationsprofilen zeigt sowohl bei der strukturierten als auch bei der permanenten Behandlung von T-Zellen und Makrophagen keine signifikante Reduktion der HIV-1 Replikation. In allen Fällen liegt die Replikationskapazität im Bereich der unbehandelten Kontrolle.

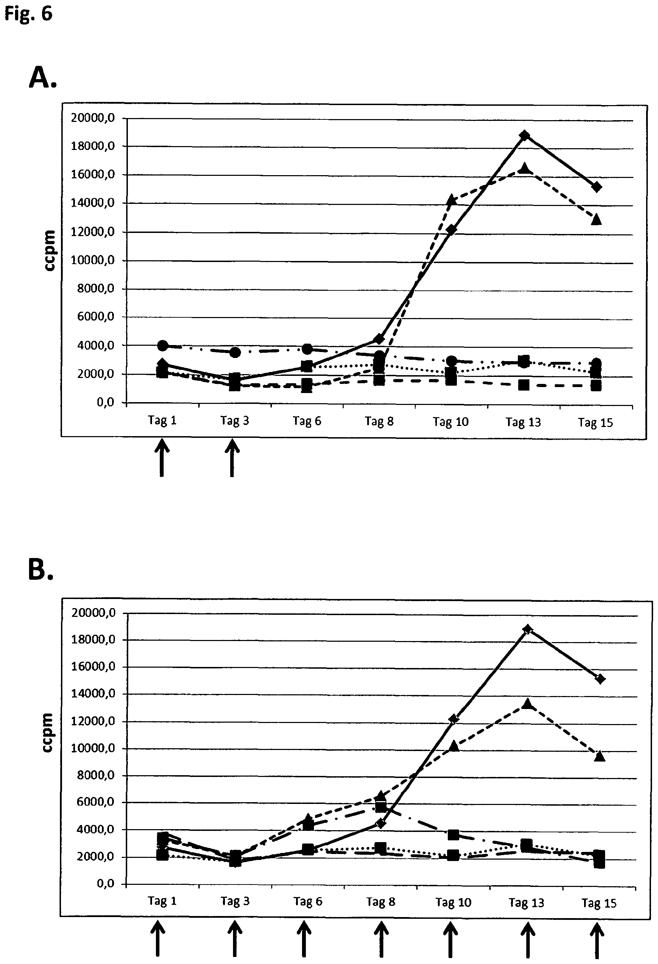

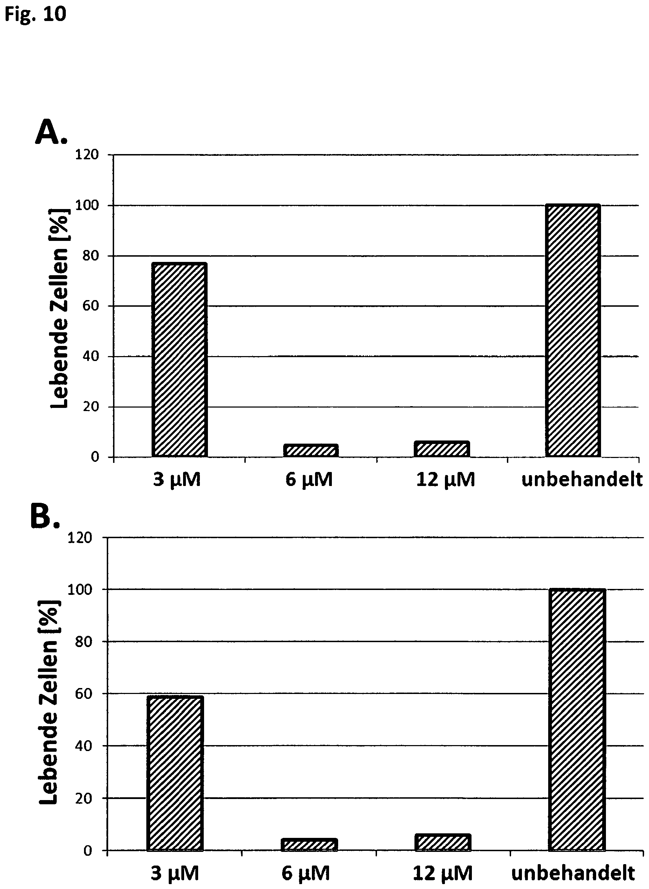

Auch die Behandlung von PBMCs mit dem DUB-Inhibitor WP1130 (Merck-Millipore) in den verwendeten, nicht zytotoxischen Konzentrationen von 3 µM und 6 µM zeigte keinen Einfluss auf die HIV-1-Replikation in T-Zellen und Makrophagen (Fig. 6A und 6B). Der leichte Rückgang in der Replikationskapazität bei permanenter Behandlung (Fig. 6B) mit 3 µM WP1130 könnte mit der hier schon auftretenden Toxizität erklärt werden (vgl. 10B). Der Verlust der Replikationskapazität bei höheren Konzentrationen von WP1130 ist auf die sehr hohe Toxizität in diesem Konzentrationsbereich zurückzuführen (Fig. 10A und 10B). Auch bei WP1130 zeigt die statistische Auswertung von 5 Replikationsprofilen sowohl bei der strukturierten als auch bei der permanenten Behandlung von T-Zellen und Makrophagen keine signifikante Reduktion der HIV-1 Replikation. In allen Fällen liegt die Replikationskapazität im Bereich der unbehandelten Kontrolle.

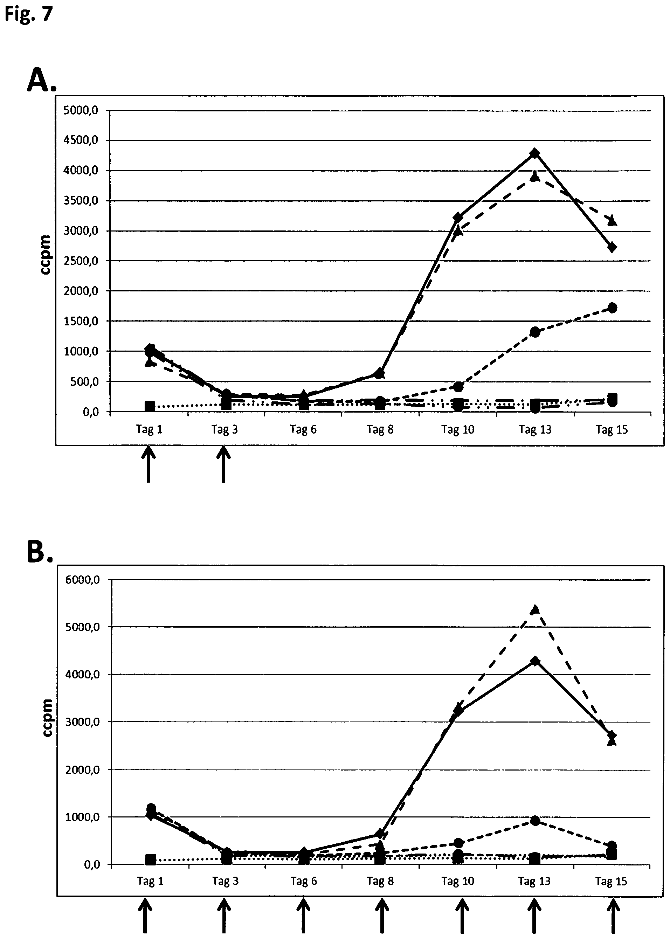

Die Behandlung mit dem DUB-Inhibitor P22077 in nicht zytotoxischen Konzentrationen von 7,5 µM, 15 µM, 30 µM und 60 µM zeigte wie bei PR-619 eine fast vollständige Hemmung der HIV-Replikation (Fig. 7A und 7B). Eine allgemeine zytotoxische Schädigung der Wirtszelle liegt nicht vor. Diese Beobachtung unterstreicht den spezifischen Effekt der DUB-Inhibitoren auf die HIV-1- Replikation.

Zusammengefasst kann aus den Ergebnissen der Beispiele 2 und 3 geschlossen werden, dass der DUB-Inhibitor PR-619 sehr spezifisch die HIV-1-Replikation hemmen kann und diese Hemmung offensichtlich nicht mit allen DUB-Inhibitoren erreicht werden kann.

Aus den spezifischen Inhibitionsprofilen der verwendeten DUB-Inhibitoren P22077, PR-619, P005091 und WP1130 (siehe oben) kann man schließen, dass der gemeinsame Wirkmechanismus die Hemmung des Enzyms USP47 sein muss. Es ist das einzige DUB-Protein, welches sowohl durch P22077 als auch durch PR-619, nicht aber durch P005091 und WP1130 gehemmt werden kann. Daraus lässt sich schlussfolgern, dass USP47 für die HIV-1-Replikation essentiell ist.

### Beispiel 4: Untersuchungen zur Zytotoxizität von PR-619, P005091 und WP1130

Um die Frage zu klären, ob PR-619 in den obigen Systemen einen zelltoxischen Effekt auslöst, wurden nicht infizierte PBMC-Kulturen parallel zu den Replikationsstudien mit den gleichen Konzentrationen an PR-619 behandelt. Die Toxizität wurde mittels WST-Assay bestimmt. Dabei bewirken viable Zellen mit einem intakten mitochondrialen Succinat-Tetrazolium-Dehydrogenase System eine enzymatische Umsetzung des schwach rot gefärbten Tetrazoliumsalzes WST-1 (4-[3-(4-lodophenyl)-2-(4-nitrophenyl)-2H-5-tetrazolio]-1,3-Benzol-Disulfonat) in das dunkelrote Formazan. Dieser Farbumschlag kann in einem Spektralphotometer photometrisch gemessen werden. Der WST-Assay stellt damit eine sehr sensitive Methode dar, um die Toxizität von Substanzen auf den Zellmetabolismus zu messen.

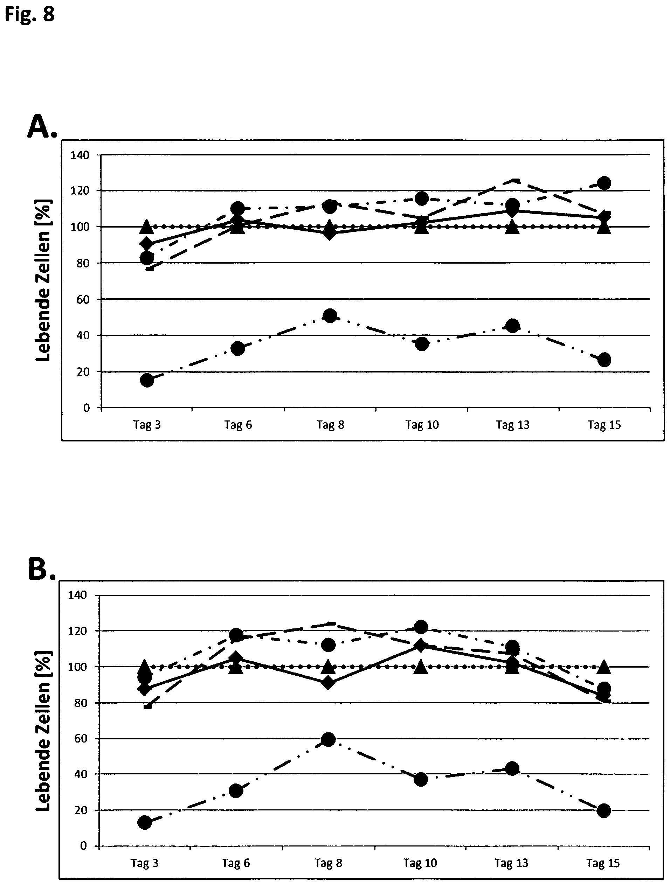

In Fig. 8A ("strukturierte Behandlung") und 8B ("permanente Behandlung") ist jeweils der Prozentsatz toter Zellen im Vergleich zu den unbehandelten Zellen (der Wert der unbehandelten Zellen wurde jeweils 100% gesetzt) dargestellt.

Es zeigte sich, dass PR-619 in antiviral wirkenden Konzentrationen im Beobachtungszeitraum von 15 Tagen keinen signifikanten toxischen Effekt aufweist. PR-619 zeigte erst ab einer Konzentration von 28 µM (im Unterschied dazu liegt die antivirale Wirksamkeit bereits bei 3,5 µM) einen deutlich toxischen Effekt.

Somit kann festgestellt werden, dass der antivirale Effekt von PR-619 nicht auf unspezifischen zytotoxischen Effekten beruht.

Vergleichende Experimente wurden mit P005091 und WP1130 durchgeführt. Hierzu wurde die Toxizität dieser beiden DUB-Inhibitoren an Tag 13 nach Behandlung von PBMC-Zellen mittels WST-Assay bestimmt. Es wurde jeweils der Prozentsatz toter Zellen im Vergleich zur unbehandelten Kontrolle (100%) dargestellt.

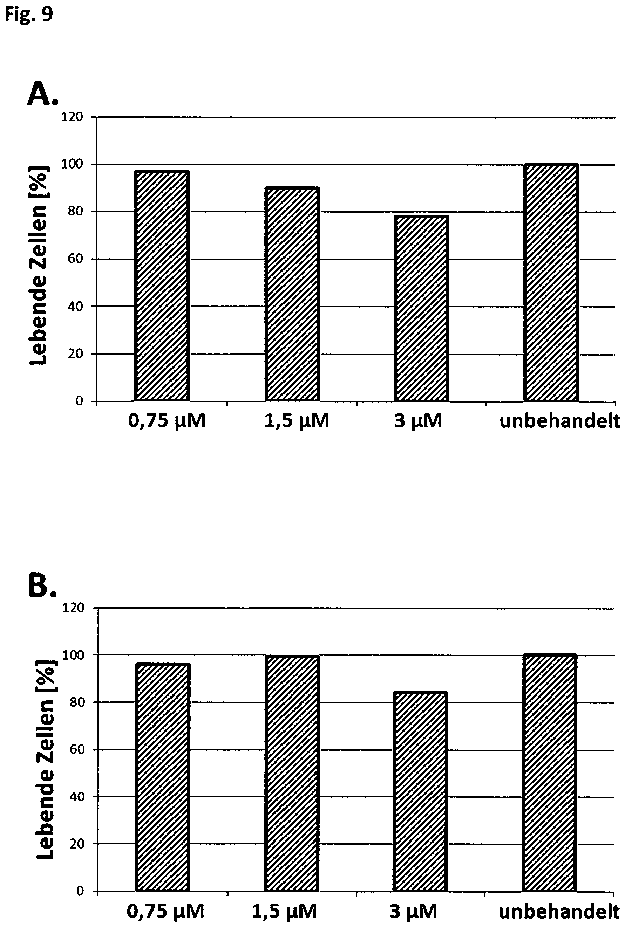

Hierbei zeigte sich, dass der DUB-Inhibitor P005091 in dem eingesetzten Konzentrationsbereich von 0.75 - 3 µM keine Toxizität aufwies (Fig. 9A und 9B). Nach Behandlung mit dem DUB-Inhibitor WP1130 hingegen zeigte sich bei permanenter Behandlung bereits in der niedrigsten eingesetzten Konzentration von 3 µM ein Rückgang der lebenden Zellen um 40%. Eine Konzentration >3 µM führte zum vollständigen Absterben der Zellen (Fig. 10A und 10B).

### Beispiel 5: PR-619 erhöht dosisabhängig die MHC-I-Antigenpräsentation von viralen Strukturproteinen

Aus vorhergehenden Untersuchungen der Anmelder ist bekannt, dass die Polyubiquitinylierung von Gag zu einem verstärkten Eintritt in das UPS und folglich zu einer erhöhten MHC-I-Antigenpräsentation führt. Um zu untersuchen, ob der erfindungsgemäße Einsatz von PR-619 die MHC-I Antigenpräsentation und dadurch die Immunogenität von HIV-1-Gag-Proteinen bezüglich der CD8⁺ T-Zell-Antwort steigert, wurde die MHC-I-Antigenpräsentation von Gag-abgeleiteten Epitopen ermittelt.

Da es keine Antikörper gibt, die an humane MHC-I-Moleküle gebundene Gag-Epitope detektieren können, wurde die aus Ovalbumin stammende Sequenz SIINFEKL (SL) als ein Modell-Epitop in die nicht-strukturierte SP1-Spacer-Region von Gag eingebracht. Für die Messung der MHC-I-Antigenpräsentation wurden HeLa-Zellen, die den murinen SLbindenden MHC-I-Allotyp H2-K_{b} (HeLa-K_{b}) (Porgador et al., 1997, Immunity 6(6), 715-726) konstitutiv exprimieren, mit Expressionsplasmiden transfiziert, welche für Gag-SL-Proteine kodieren. In durchflusszytometrischen Untersuchungen wurde der monoklonale Antikörper (mAK) 25D1.16 verwendet, der spezifisch SL im Komplex mit H2-K_{b} erkennt.

24 h nach Transfektion wurden die Zellen mit verschiedenen Konzentrationen des DUB-Inhibitors PR-619 (7 und 14 µM) über Nacht inkubiert.

Daraufhin wurde ein *acid wash* (saurer Waschpuffer pH 3) durchgeführt, um die H2-K_{b}-SL-Komplexe von der Zelloberfläche zu entfernen. Die Zellen wurden daraufhin für 4 h mit und ohne PR-619 bei 37°C inkubiert und anschließend die Neubeladung der H2-K_{b}-SL-Komplexe an der Zelloberfläche unter Inhibition mit PR-619 mittels FACS-Analysen mit dem monoklonalen AK 25D1.16 gemessen. Um mögliche Unterschiede im Expressionslevel von Gag-SL zu kompensieren, wurde die mittlere Fluoreszenzintensität (MFI) der 25D1.16-Färbung auf die MFI des intrazellulären Gag-Signals normalisiert.

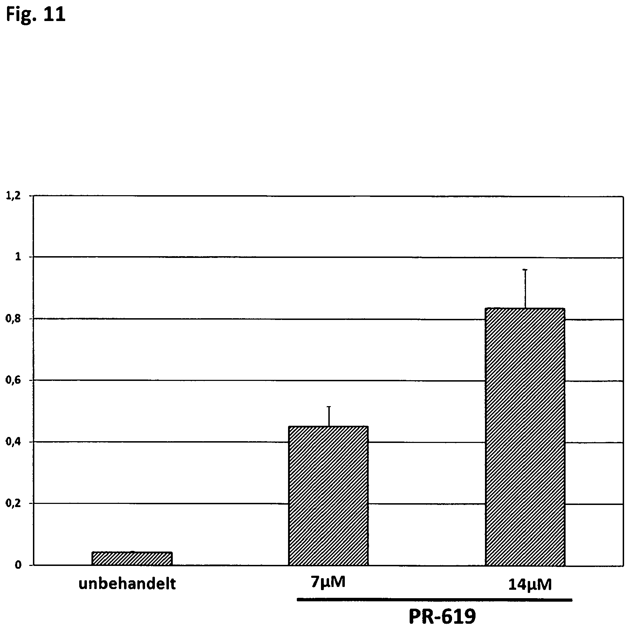

Dabei ist deutlich zu erkennen, dass die Behandlung der Zellen mit PR-619 dosisabhängig die MHC-I-Antigenpräsentation von Gag-abgeleiteten Epitopen um das 6-fache erhöht (Fig. 11).

Zusammengefasst kann festgestellt werden, dass der erfindungsgemäße Einsatz von PR-619 nicht nur die HIV-1-Replikation hemmt, sondern gleichzeitig auch die Immunogenität von HIV-1-Strukturproteinen deutlich steigert.

### Beispiel 6: Der DUB-Inhibitor PR-619 hemmt in Kombination mit dem Proteasom-Inhibitor Bortezomib synergistisch die HIV-1 Replikation in T-Zellen und Makrophagen

Um die Frage zu klären, ob die kombinierte, permanente Behandlung mit PR-619 und dem Proteasom-Inhibitor Bortezomib (Velcade®) einen synergistischen Effekt in der Hemmung der Replikationskapazität von HIV-1 in T-Zellen und Makrophagen ausübt, wurde eine Kreuztitration mit den beiden Substanzen durchgeführt (in Analogie zu Beispiel 2 und 3). Dabei wurden PBMC-Kulturen nach Infektion mit dem Virusstamm HIV-1_{NL4-3} (X4-troph) bzw. HIV-1_{NL4-3} (R5-troph) zunächst nur mit dem DUB-Inhibitor PR-619 oder dem Proteasom-Inhibitor Bortezomib in nicht toxischen, aufsteigenden Konzentrationen (PR-619: 0,3 µM, 0,9 µM, 2,7 µM und 8 µM; Bortezomib: 0,6 nM, 1,5 nM, 5,3 nM und 16 nM) permanent behandelt. In einem weiteren Versuchsansatz wurde eine Kreuztitration mit PR-619 und Bortezomib durchgeführt. Dabei wurden die Zellen konstant mit 0,6 nM Bortezomib und aufsteigenden Konzentrationen von PR-619 (0,3 µM, 0,9 µM, 2,7 µM und 8 µM) permanent behandelt. Die Versuche wurden wie unter Beispiel 2 beschrieben durchgeführt.

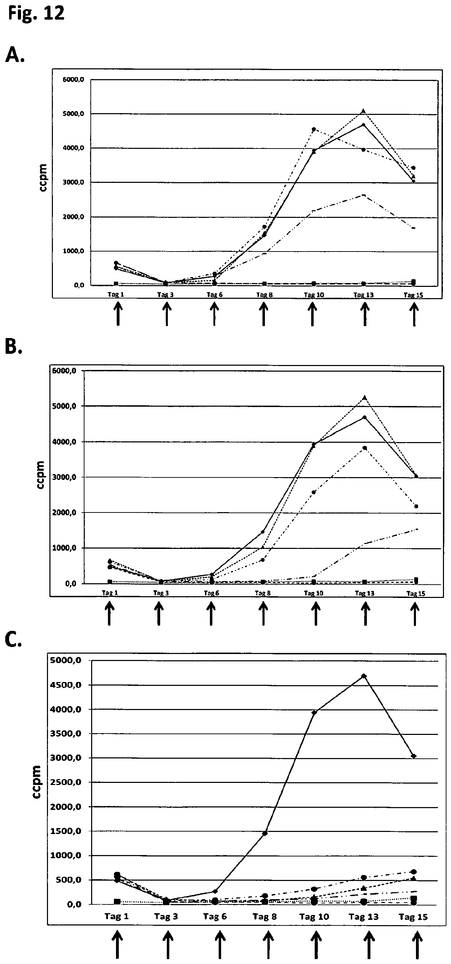

Dabei zeigte sich, dass der Proteasom-Inhibitor Bortezomib ab einer Konzentration von 5,3 nM die HIV-1 Replikation sowohl in T-Zellen als auch in Makrophagen hemmt. Fig. 12A zeigt das Ergebnis in T-Zellen. Der DUB-Inhibitor PR-619 ist ab einer Konzentration von 2,7 µM in der Lage, die Replikation von HIV-1 zu hemmen (Fig.12B).

Nach Behandlung der PBMC-Kulturen mit einer konstanten Konzentration von 0,6 nM Bortezomib und aufsteigenden Konzentrationen von PR-619 (0,3 µM, 0,9 µM, 2,7 µM und 8 µM) zeigte sich bereits bei den niedrigsten eingesetzten Konzentrationen von 0,3 µM PR-619 und 0,6 nM Bortezomib eine vollständige Hemmung der Replikation. Dies war sowohl in T-Zellen (Fig. 12C) als auch in Makrophagen zu beobachten. In diesen niedrigen Konzentrationen waren die Substanzen, einzeln eingesetzt, nicht in der Lage, die HIV-1 Replikationskapazität herabzusetzen.

Die statistische Auswertung der HIV-1 Replikationskapazität nach Infektion von PBMCs aus 4 verschiedenen Spendern ergab, dass der kombinierte Einsatz von konstant 0,6 nM Bortezomib und 0,3 µM PR-619 die HIV-1 Replikation um 55 % (+/- 13 %), 0,9 µM PR-619 um ca. 72 % (+/- 9 %) und 2,7 µM PR-619 um 90 % (+/- 4%) hemmt.

Nach Infektion von Makrophagen aus 5 verschiedenen Spendern zeigte die statistische Auswertung der HIV-1 Replikationskapazität, dass der kombinierte Einsatz von konstant 0,6 nM Bortezomib und 0,3 µM PR-619 die HIV-1 Replikation um 45 % (+/- 14 %), 0,9 µM PR-619 um ca. 73 % (+/- 9 %) und 2,7 µM PR-619 um 87 % (+/- 4%) hemmt.

Erfindungsgemäß zeigen diese Ergebnisse, dass der kombinierte Einsatz von PR-619 und Bortezomib in jeweils sehr niedrigen Konzentrationen eine supraadditive synergistische Hemmung der HIV-1 Replikation in T-Zellen und in Makrophagen aufweist.

### Beispiel 7: Untersuchungen zur Zytotoxizität von PR-619, Bortezomib und der Kombination der beiden Substanzen bei permanenter Behandlung

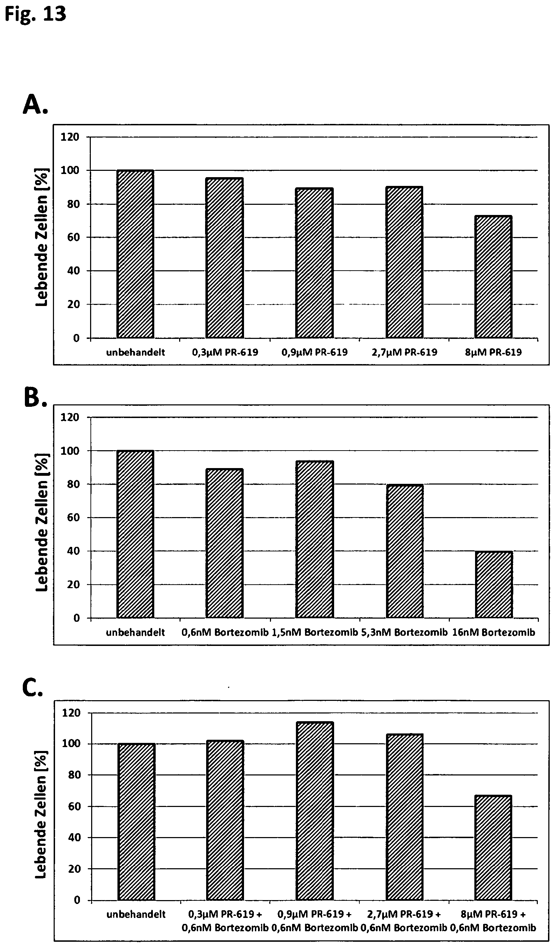

Um die Frage zu klären, ob PR-619 oder Bortezomib einzeln eingesetzt, oder bei kombinierter Behandlung in den obigen Systemen einen zelltoxischen Effekt auslösen, wurden nicht infizierte PBMC-Kulturen parallel zu den Replikationsstudien (unter Beispiel 2 beschrieben) mit den gleichen Konzentrationen von PR-619, Bortezomib und der Kombination beider Substanzen behandelt. Die Toxizität wurde mittels WST-Assay, wie in Beispiel 4 beschrieben, bestimmt. In Fig. 13 ist jeweils der Prozentsatz toter Zellen im Vergleich zu den unbehandelten Zellen (der Wert der unbehandelten Zellen wurde jeweils 100% gesetzt) dargestellt.

Es zeigte sich, dass PR-619 in antiretroviral wirkenden Konzentrationen im Beobachtungszeitraum von 15 Tagen keinen signifikanten toxischen Effekt aufweist. PR-619 zeigte auch bei der höchsten eingesetzten Konzentration von 8 µM keinen deutlich toxischen Effekt (Fig.13A).

Vergleichende Experimente wurden mit dem Proteasom-Inhibitor Bortezomib durchgeführt. Hierbei zeigte sich, dass Bortezomib in einem Konzentrationsbereich von 0.6 - 5.3 nM keine Toxizität aufwies (Fig. 13B). Ab einer Konzentration von 16 nM zeigte sich ein Rückgang der lebenden Zellen um 60%.

Die Kombination der beiden Substanzen wies in antiretroviral wirkenden Konzentrationen keine Toxizität (0,6 nM Bortezomib + 0,3 µM, 0,9 µM, 2,7 µM PR-619) auf (Fig.13C). Erst ab einer Konzentration von 8 µM PR-619 und 0,6 nM Bortezomib war ein Rückgang der lebenden Zellen um 40% zu beobachten.

Somit kann festgestellt werden, dass der antiretrovirale Effekt der Kombinationsbehandlung von PR-619 und Bortezomib nicht auf unspezifischen zytotoxischen Effekten beruht, sondern auf einen spezifischen, synergistischen Wirkmechanismus zurückzuführen ist.

### Beispiel 8: Der DUB-Inhibitor PR-619 hemmt in Kombination mit dem Proteasom-Inhibitor PR-957 synergistisch die HIV-1 Replikation im HLAC-System

Um die Frage zu klären, ob die kombinierte, permanente Behandlung auch mit anderen, sehr spezifischen Proteasom-Inhibitoren einen synergistischen Effekt in der Hemmung der Replikationskapazität von HIV-1 zeigt, wurde der spezifische Inhibitor des Immunoproteasoms PR-957 verwendet, der im Gegensatz zu Bortezomib zur Gruppe der Epoxyketone gehört. Es wurden Kreuztitrationen mit den beiden Substanzen durchgeführt (in Analogie zu Beispiel 6). Dabei wurden PBMC-Kulturen nach Infektion mit dem Virusstamm HIV-1_{NL4-3} X4-troph zunächst nur mit dem DUB-Inhibitor PR-619 oder dem Immunoproteasom-Inhibitor PR-957 in nicht toxischen, aufsteigenden Konzentrationen (PR-619: 0,3 µM, 0,9 µM, 2,7 µM und 8 µM); PR-957: 20 nM, 40 nM, 80 nM und 160 nM) permanent behandelt. In einem weiteren Versuchsansatz wurde eine Kreuztitration mit PR-619 und PR-957 durchgeführt. Dabei wurden die Zellen konstant mit 40 nM PR-957 und aufsteigenden Konzentrationen von PR-619 (0,3 µM, 0,9 µM, 2,7 µM und 8 µM) permanent behandelt. Die Versuche wurden wie unter Beispiel 6 beschrieben durchgeführt.

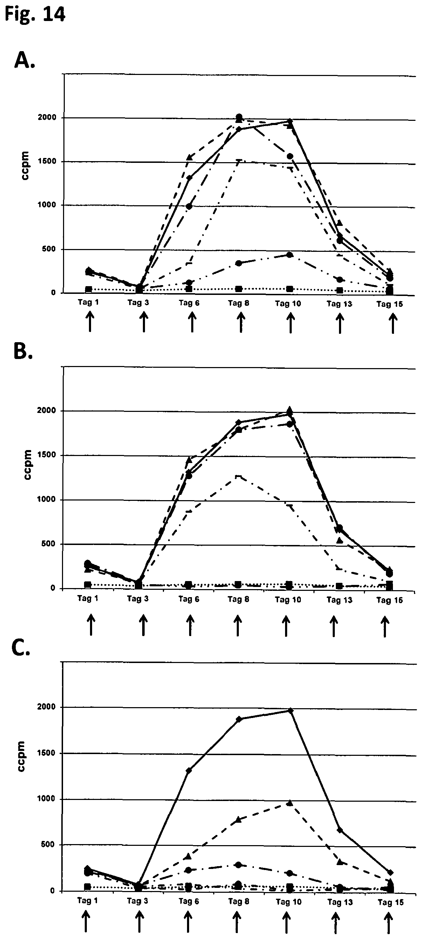

Dabei zeigte sich, dass der Immunoproteasom-Inhibitor PR-957 ab einer Konzentration von 80 nM die HIV-1 Replikation hemmt (Fig. 14A).

Der DUB-Inhibitor PR-619 ist ab einer Konzentration von 2,7 µM in der Lage, die Replikation von HIV-1 zu hemmen (Fig.14B).

Nach Behandlung der PBMC-Kulturen mit einer konstanten Konzentration von 40 nM PR-957 und aufsteigenden Konzentrationen von PR-619 (0,3 µM, 0,9 µM), 2,7 µM und 8 µM) zeigte sich bereits bei 0,9 µM PR-619 und 40 nM PR-957 eine vollständige Hemmung der Replikation (Fig. 14C). In diesen niedrigen Konzentrationen waren die Substanzen, einzeln eingesetzt, nicht in der Lage, die HIV-1 Replikationskapazität herabzusetzen.

Die statistische Auswertung der HIV-1 Replikationskapazität nach Infektion von PBMCs ergab, dass der kombinierte Einsatz von konstant 40 nM PR-957und 0,3 µM PR-619 die HIV-1 Replikation um 56 %, 0,9 µM PR-619 um 84% und 2,7 µM PR-619 um 94 % hemmt.

Diese Ergebnisse zeigen, dass der kombinierte Einsatz von PR-619 und PR-957 in jeweils sehr niedrigen Konzentrationen eine supraadditive synergistische Hemmung der HIV-1 Replikation bewirkt.

### Beispiel 9: Untersuchungen zur Zytotoxizität von PR-619, PR-957 und der Kombination der beiden Substanzen bei permanenter Behandlung

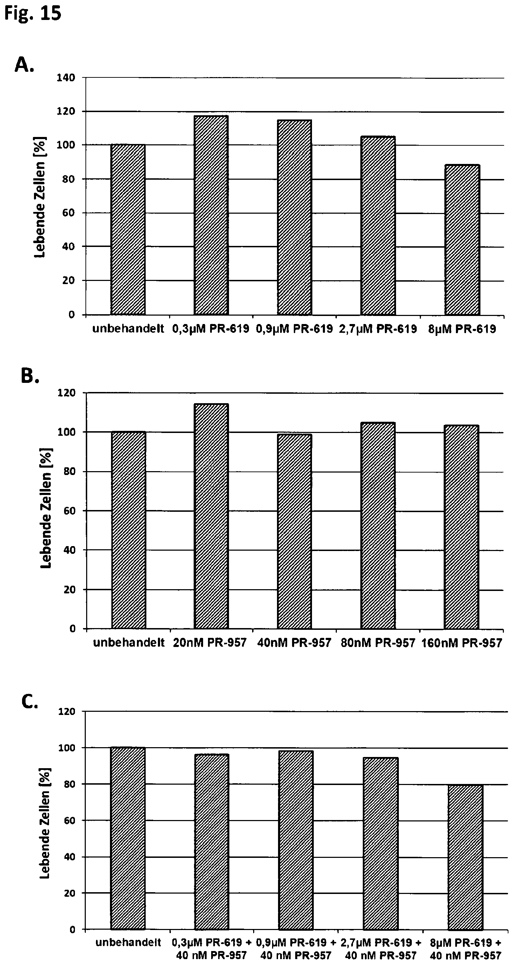

Um die Frage zu klären, ob PR-619 oder PR-957 einzeln eingesetzt, oder bei kombinierter Behandlung in den obigen Systemen einen zelltoxischen Effekt auslösen, wurden infizierte PBMC-Kulturen parallel zu den Replikationsstudien (unter Beispiel 8 beschrieben) mit den gleichen Konzentrationen von PR-619, PR-957 und der Kombination beider Substanzen behandelt. Die Toxizität wurde mittels WST-Assay, wie in Beispiel 4 beschrieben, bestimmt. In Fig. 15 ist jeweils der Prozentsatz toter Zellen im Vergleich zu den unbehandelten Zellen (der Wert der unbehandelten Zellen wurde jeweils 100% gesetzt) dargestellt.

Es zeigte sich, dass PR-619 in antiretroviral wirkenden Konzentrationen im Beobachtungszeitraum von 15 Tagen keinen signifikanten toxischen Effekt aufweist. PR-619 zeigte auch bei der höchsten eingesetzten Konzentration von 8 µM keinen deutlich toxischen Effekt (Fig.15A).

Vergleichende Experimente wurden mit dem Immunoproteasom-Inhibitor PR-957 durchgeführt. Hierbei zeigte sich, dass PR-957 im untersuchten Konzentrationsbereich von 20 - 160 nM keine Toxizität aufwies (Fig. 15B).

Die Kombination der beiden Substanzen wies in antiretroviral wirkenden Konzentrationen keine Toxizität (40 nM PR-957 + 0,3 µM, 0,9 µM, 2,7 µM PR-619) auf (Fig.15C). Erst ab einer Konzentration von 8 µM PR-619 und 40 nM PR-957 war ein Rückgang der lebenden Zellen um 20% zu beobachten.

Somit kann festgestellt werden, dass der antiretrovirale Effekt der Kombinationsbehandlung von PR-619 und PR-957 nicht auf unspezifischen zytotoxischen Effekten beruht, sondern auf einen spezifischen, synergistischen Wirkmechanismus zurückzuführen ist.

Beispiel 10: Der DUB-Inhibitor PR-619 hemmt konzentrationsabhängig die HIV-1-Replikation im *human lymphoid aggregate culture* (HLAC) ex *vivo*-System, ohne die Vitalität der Wirtszelle zu beeinflussen.

Um die Relevanz der in Beispiel 2 beobachteten Hemmung der HIV-Replikation unter Behandlung mit PR-619 weiter zu untermauern, wurde erfindungsgemäß humanes, ex *vivo* kultiviertes Tonsillengewebe mit dem Virusstamm HIV-1_{NL4-3} (X4-troph) bzw. HIV-1_{NL4-3}(R5-troph) infiziert. Die Versuche wurden wie unter Beispiel 2 beschrieben durchgeführt.

Dabei zeigt sich in T-Zellen und Makrophagen des HLAC-Systems eine dosisabhängige Inhibierung der HIV-1-Replikation. Während 1,25 µM PR-619 keinen hemmenden Effekt auf die HIV-1-Replikation ausübt, bewirkt der Einsatz von 2,5 µM, 5 µM und 10 µM PR-619 eine deutliche bzw. vollständige Hemmung der Replikation.

Die statistische Auswertung von 3 Replikationsprofilen zeigt bei permanenter Behandlung mit PR-619 eine signifikante, dosisabhängige Reduktion der HIV-1 Replikationskapazität in T-Zellen und Makrophagen. Bei einer Konzentration von 2,5 µM PR-619 war die Replikationskapazität um 40 % (+/- 9 %), bei 5 µM um 86 % (+/- 2%) und bei 10 µM vollständig gehemmt.

Somit führt der erfindungsgemäße Einsatz des DUB-Inhibitors PR-619 im HLAC-System dosisabhängig zu einer vollständigen Hemmung der HIV-1- Replikation.

### Beispiel 11: Untersuchungen zur Zytotoxizität von PR-619 bei permanenter Behandlung von ex vivo kultiviertem Tonsillengewebe (HLAC)

Um die Frage zu klären, ob PR-619 in den obigen Systemen (Beispiel 10) einen zelltoxischen Effekt auslöst, wurden infizierte HLAC-Kulturen aus ex *vivo* kultiviertem Tonsillengewebe parallel zu den Replikationsstudien (unter Beispiel 10 beschrieben) mit den gleichen Konzentrationen von PR-619 behandelt. Die Toxizität wurde mittels WST-Assay, wie in Beispiel 4 beschrieben, bestimmt.

Es zeigte sich, dass PR-619 in allen eingesetzten, antiretroviral wirkenden Konzentrationen im Beobachtungszeitraum von 15 Tagen keinen signifikanten toxischen Effekt aufweist. Selbst bei der höchsten Konzentration von 10 µM PR-619 waren keine toxischen Effekte zu beobachten.

Somit kann festgestellt werden, dass der antiretrovirale Effekt nach Behandlung von ex *vivo* HLAC-Kulturen mit PR-619 nicht auf unspezifischen zytotoxischen Effekten beruht, sondern auf einen spezifischen Wirkmechanismus zurückzuführen ist.

### Figuren

Fig. 1: Schema der Prozessierung von Pr55Gag
Fig. 2: Western Blots der Pr55Gag-Prozessierungprodukte nach Gabe von PR-619
Fig. 3: Densitometrische Auswertung der VLP-Fraktionen aus Fig. 2
Fig. 4: "Strukturierte Behandlung" (A) und "Permanente Behandlung" (B) von HIV-1-infizierten T-Zellen mit verschiedenen Konzentrationen von PR-619

| | | | | | | | |
|---|---|---|---|---|---|---|---|
| 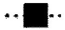 | Nicht infiziert | 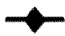 | unbehandelt | | | | |
| 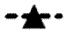 | 3,5 µM | 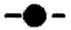 | 7 µM | 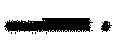 | 14 µM | 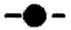 | 28 µM |
| | PR-619 | | PR-619 | | PR-619 | | PR-619 |

Fig. 5: "Strukturierte Behandlung" (A) und "Permanente Behandlung" (B) von HIV-1-infizierten T-Zellen mit verschiedenen Konzentrationen von P005091

| | | | | | |
|---|---|---|---|---|---|
| 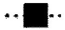 | Nicht infiziert | 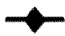 unbehandelt | | | |
| 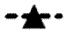 | 0,75 µM | 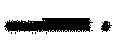 | 1,5 µM | 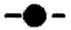 | 3 µM |
| | P005091 | | P005091 | | P005091 |

Fig. 6: "Strukturierte Behandlung" (A) und "Permanente Behandlung" (B) von HIV-1-infizierten T-Zellen mit verschiedenen Konzentrationen von WP1130

| | | | | | |
|---|---|---|---|---|---|
| 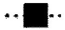 | Nicht infiziert | 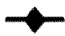 | unbehandelt | | |
| 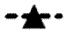 | 3 µM | 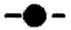 | 6 µM | 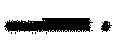 | 12 µM |
| | WP1130 | | WP1130 | | WP1130 |

Fig. 7: "Strukturierte Behandlung" (A) und "Permanente Behandlung" (B) von HIV-1-infizierten T-Zellen mit verschiedenen Konzentrationen von P22077

| | | | | | | | |
|---|---|---|---|---|---|---|---|
| 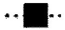 | Nicht infiziert | 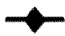 | unbehandelt | | | | |
| 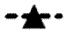 | 7,5 µM | 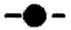 | 15 µM | 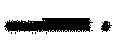 | 30 µM | 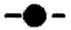 | 60 µM |
| | P22077 | | P22077 | | P22077 | | P22077 |

Fig. 8: Prozentsatz lebender Zellen nach Behandlung mit verschiedenen Konzentrationen von PR-619 bei "Strukturierter Behandlung" (A) und "Permanenter Behandlung" (B) über einen Zeitraum von 15 Tagen

| | | | | | | | |
|---|---|---|---|---|---|---|---|
| 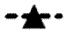 | unbehandelt | | | | | | |
| 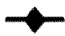 | 3,5 µM PR-619 | 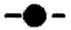 | 7 µM PR-619 | 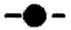 | 14 µM PR-619 | 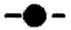 | 28 µM PR-619 |

Fig. 9: Prozentsatz lebender Zellen nach Behandlung mit verschiedenen Konzentrationen von P005091 bei "Strukturierter Behandlung" (A) und "Permanenter Behandlung" (B)
Fig. 10: Prozentsatz lebender Zellen nach Behandlung mit verschiedenen Konzentrationen von WP1130 bei "Strukturierter Behandlung" (A) und "Permanenter Behandlung" (B)
Fig. 11: Dosisabhängige Erhöhung der MHC-I-Antigenpräsentation nach Zugabe des DUB-Inhibitors PR-619
Fig. 12: "Permanente Behandlung" von HIV-1-infizierten T-Zellen mit verschiedenen Konzentrationen von Bortezomib (A), PR-619 (B) und der Kombination der beiden Substanzen (C)

| | | | | | | | |
|---|---|---|---|---|---|---|---|
| 12A: | | | | | | | |
| 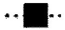 | Nicht infiziert | 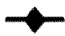 | unbehandelt | | | | |
| 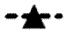 | 0,6 nM Bortezomib | 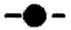 | 1,5 nM Bortezomib | 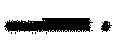 | 5,3 nM Bortezomib | 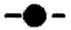 | 16 nM Bortezomib |
| 12B: | | | | | | | |
| Nicht infiziert unbehandelt | | | | | | | |
| 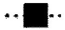 | 0,3 µM PR-619 | 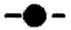 | 0,9 µM PR-619 | 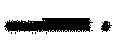 | 2,7 µM PR-619 | 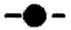 | 8 µM PR-619 |
| 12C: | | | | | | | |
| 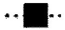 | Nicht infiziert | 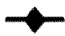 | unbehandelt | | | | |
| 0,3 + | µM PR-619 0,6 nM Bortezomib | 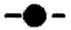 | 0,9 µM PR-619 + 0,6 Bortezomib | 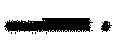 | 2,7 µM PR-619 + 0,6 nM Bortezomib | 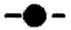 | 8 µM PR-619 + 0,6 nM Bortezomib |

Fig. 13: Prozentsatz lebender Zellen nach Behandlung mit verschiedenen Konzentrationen von PR-619 (A), Bortezomib (B) und der Kombination der beiden Substanzen (C) bei "Permanenter Behandlung"
Fig. 14: "Permanente Behandlung" von HIV-1-infizierten T-Zellen mit verschiedenen Konzentrationen von PR-957 (A), PR-619 (B) und der Kombination der beiden Substanzen (C) 14A:

| | | | | | | | |
|---|---|---|---|---|---|---|---|
| 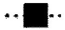 | Nicht infiziert | 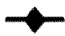 | unbehandelt 5 | | | | |
| 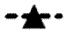 | 20 nM PR-957 | 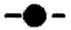 | 40 nM PR-957 | 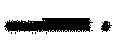 | 80 nM PR-957 | 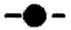 | 160 nM PR-957 |
| | 14B: | | | | | | |
| 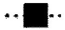 | Nicht infiziert | 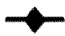 | unbehandelt | | | | |
| | 0,3 µM PR-619 | 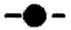 | 0,9 µM PR-619 | 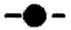 | 2,7 µM PR-619 | 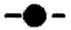 | 8 µM PR-619 10 |
| | 14C: | | | | | | |
| 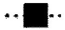 | Nicht infiziert | 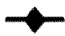 | unbehandelt | | | | |
| 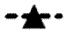 | 0,3 µM PR-619 + 40nM PR-957 | 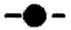 | 0,9 µM PR-619 + 40nM PR-957 | 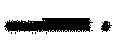 | 2,7 µM PR-619 + 40nM PR-957 | 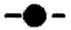 | 8 µM PR-619 + 40nM PR-957 |

- Fig. 15:: Prozentsatz lebender Zellen nach Behandlung mit verschiedenen Konzentrationen von PR-619 (A), PR-957 (B) und der Kombination der beiden Substanzen (C) bei "Permanenter Behandlung"

## Patentansprüche

1. Verbindungen gemäß Formel (I) zur Verwendung bei der Behandlung von retroviralen Infektionen sowie zur Prophylaxe vor der Manifestation retroviraler Erkrankungen, 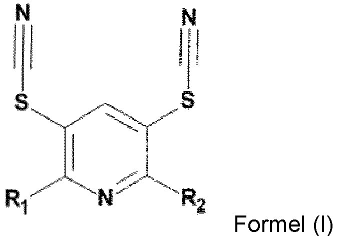
wobei R₁ und R₂ jeweils unabhängig voneinander für H, -OH, -NHR₃, -NR₃R₄, einen substituierten oder nicht-substituierten, linearen oder verzweigten Alkyl-Rest mit 1 bis 3 Kohlenstoffatomen, -CO-OCH₃, -CO-OC₂H₅, -CO-NH₂, -NH₂, -NO₂, -Cl, -Br, -F, -SO₂H stehen; und
R₃ und R₄ jeweils unabhängig voneinander für -OH, -CH₃, -C₂H₅, -CH₂OH, -CHO, - COOH, -CO-CH3, -CO-NH₂ stehen
sowie ihre pharmazeutisch verträglichen Salze, Hydrate und Solvate.

2. Verbindungen zur Verwendung gemäß Anspruch 1, wobei die retrovirale Infektion aus der Gruppe bestehend aus HIV-1-Infektion, HIV-2-Infektion, AIDS, FIV-Infektion und SIV-Infektion ausgewählt wird.

3. Verbindung zur Verwendung gemäß einem der Ansprüche 1 oder 2, wobei die Verbindung 2,6-Diaminopyridin-3,5-bis(thiocyanat) ist.

4. Verbindungen wie in Anspruch 1 definiert zusammen mit mindestens einem weiteren Wirkstoff, zur Verwendung gemäß einem der Ansprüche 1 bis 3, wobei der besagte weitere Wirkstoff aus einer Gruppe umfassend Proteasom-Inhibitoren, Reverse Transkriptase-Inhibitoren, Integrase-Inhibitoren, HIV-Protease-Inhibitoren, Entry-Inhibitoren, HIV-Vakzine, Virostatika und Immunstimulanzien ausgewählt wird.

5. Wirkstoffkombination zur Verwendung gemäß Anspruch 4, wobei der besagte Proteasom-Inhibitor Bortezomib oder PR-957 ist.

6. Verbindung zur Verwendung gemäß Anspruch 1 oder 3 oder Wirkstoffkombination zur Verwendung gemäß Anspruch 4 oder 5 in einer Formulierung zur oralen Verabreichung.

7. Verbindung zur Verwendung gemäß Anspruch 1 oder 3 oder Wirkstoffkombination zur Verwendung gemäß Anspruch 4 oder 5 in einer Formulierung als Lyophilisat oder einer flüssigen Formulierung.

8. Wirkstoffkombination von 2,6-Diaminopyridin-3,5-bis(thiocyanat) und einem Proteasom-Inhibitor, sowie ihrer jeweiligen pharmazeutisch verträglichen Salze, Solvate und Hydrate.

9. Wirkstoffkombination gemäß Anspruch 8, wobei der Proteasom-Inhibitor Bortezomib ist.

10. Wirkstoffkombination gemäß Anspruch 8, wobei der Proteasom-Inhibitor PR-957 ist.

11. Wirkstoffkombination gemäß einem der Ansprüche 8 bis 10 zur Verwendung in der Medizin.

12. Pharmazeutische Zusammensetzung zur Verwendung bei der Behandlung von retroviralen Infektionen sowie zur Prophylaxe vor der Manifestation retroviraler Erkrankungen, die eine Verbindung wie in Anspruch 1 oder 3 definiert oder eine Kombination wie in Anspruch 4 oder 5 definiert zusammen mit mindestens einem pharmazeutisch verträglichen Trägerstoff, Hilfsstoff, Verdünnungsmittel, Kryoprotektivum und/oder Lyoprotektivum enthält.

13. Pharmazeutische Zusammensetzung zur Verwendung gemäß Anspruch 12, die zur oralen, parenteralen, topischen Verabreichung und/oder zur Verabreichung per Inhalation geeignet ist.

## Claims

1. Compounds according to Formula (I) for use in the treatment of retroviral infections as well as in the prophylaxis of the manifestation of retroviral diseases, 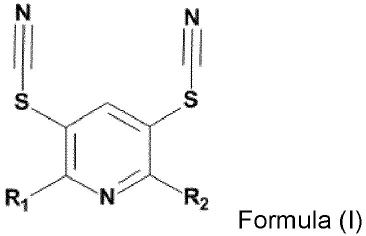
wherein R₁ and R₂ each independently from one another is -H, -OH, -NHR₃, - NR₃R₄, a substituted or non-substituted linear or ramified alkyl residue with 1 to 3 carbon atoms, -CO-OCH₃, -CO-OC₂H₅, -CO-NH₂, -NH₂, -NO₂, -CI, -Br, -F, - SO₂H; and
R₃ and R₄ each independently from one another is -OH, -CH₃, -C₂H₅, -CH₂OH, - CHO, -COOH, -CO-CH₃, -CO-NH₂,
as well as their pharmaceutically acceptable salts, hydrates and solvates.

2. Compounds for use according to claim 1, wherein the retroviral infection is selected from the group consisting of HIV-1 infection, HIV-2 infection, AIDS, FIV infection and SIV infection.

3. Compound for use according to any of claims 1 or 2, wherein the compound is 2,6-diaminopyridine-3,5-bis(thiocyanate).

4. Compounds as defined in claim 1 together with at least one further active agent for use according to one of claims 1 to 3, wherein said further active agent is selected from a group comprising proteasome inhibitors, reverse transcriptase inhibitors, integrase inhibitors, HIV protease inhibitors, entry inhibitors, HIV vaccine, virostatic agents and immunostimulatory agents.

5. Combination of active agents for use according to claim 4, wherein said proteasome inhibitor is bortezomib or PR-957.

6. Compound for use according to claim 1 or 3 or a combination of active agents according to claim 4 or 5 in a formulation for oral administration.

7. Compound for use according to claim 1 or 3 or a combination of active agents according to claim 4 or 5 in a formulation as lyophilizate or in a fluid formulation.

8. Combination of active agents of 2,6-diaminopyridine-3,5-bis(thiocyanate) and a proteasome inhibitor, as well as their respective pharmaceutically acceptable salts, solvates and hydrates.

9. Combination of active agents according to claim 8, wherein the proteasome inhibitor is bortezomib.

10. Combination of active agents according to claim 8, wherein the proteasome inhibitor is PR-957.

11. Combination of active agents according to one of claims 8 to 10 for use in medicine.

12. Pharmaceutical composition for use in the treatment of retroviral infections as well as in the prophylaxis of the manifestation of retroviral diseases containing a compound as defined in claim 1 or 3 or a combination as defined in claim 4 or 5 together with at least one pharmaceutically acceptable carrier, excipient, diluent, cryoprotectant and/or lyoprotectant.

13. Pharmaceutical composition for use according to claim 12, which is suitable for oral, parenteral, topical administration and/or for administration via inhalation.

## Revendications

1. Composés selon la formule (I) pour l'utilisation dans le traitement des infections rétrovirales ainsi que dans la prophylaxie de la manifestation des maladies rétrovirales, 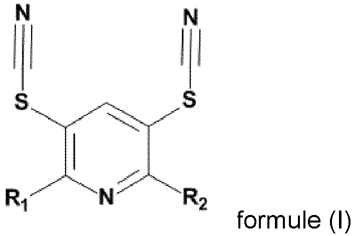
dans laquelle R₁ et R₂ représentent chacun, indépendamment un de l'autre, -H, -OH, -NHR₃, -NR₃R₄, un résidu linéaire ou ramifié, substitué ou non-substitué, comportant 1 à 3 atomes de carbone, -CO-OCH₃, -CO-OC₂H₅, -CO-NH₂, -NH₂, - NO₂, -Cl, -Br, -F, -SO₂H; et
R₃ et R₄ représentent chacun, indépendamment un de l'autre, -OH, -CH₃, -C₂H₅, -CH₂OH, -CHO, -COOH, -CO-CH₃, -CO-NH₂,
ainsi que leurs sels pharmaceutiquement acceptables, hydrates et solvates.

2. Composés pour l'utilisation selon la revendication 1, dans laquelle l'infection rétrovirale est choisie dans le groupe constitué par l'infection par le VIH-1, l'infection par le VIH-2, SIDA, l'infection par le VIF et l'infection par le VIS.

3. Composés pour l'utilisation selon l'une quelconque des revendications 1 ou 2, dans laquelle le composé est 2,6-diaminopyridine-3,5-bis(thiocyanate).

4. Composés comme définis dans la revendication 1 conjointement avec au moins un autre agent actif pour l'utilisation selon une des revendications 1 à 3, dans laquelle ledit autre agent actif est choisi dans le groupe comprenant inhibiteurs du protéasome, inhibiteurs de la transcriptase inverse, inhibiteurs de l'intégrase, inhibiteurs de la protéase de VIH, inhibiteurs d'entrée, vaccin contre le VIH, agents virostatiques et agents immunostimulantes.

5. Combination d'agents actifs pour l'utilisation selon la revendication 4, dans laquelle ledit inhibiteur du protéasome est bortézomib ou PR-957.

6. Composés pour l'utilisation selon la revendication 1 ou 3 combination d'agents actifs selon la revendication 4 ou 5 dans une formulation pour l'administration orale.

7. Composés pour l'utilisation selon la revendication 1 ou 3 combination d'agents actifs selon la revendication 4 ou 5 dans une formulation comme lyophilisat ou dans une formulation fluide.

8. Combination d'agents actifs de 2,6-diaminopyridine-3,5-bis(thiocyanate) et d'un inhibiteur du protéasome, ainsi que leurs sels pharmaceutiquement acceptables, solvates et hydrates respectifs.

9. Combination d'agents actifs selon la revendication 8, dans laquelle l'inhibiteur du protéasome est bortézomib.

10. Combination d'agents actifs selon la revendication 8, dans laquelle l'inhibiteur du protéasome est PR-957.

11. Combination d'agents actifs selon l'une quelconque des revendications 8 à 10 pour l'utilisation dans la médicine.

12. Composition pharmaceutique pour l'utilisation dans le traitement des infections rétrovirales ainsi que dans la prophylaxie de la manifestation des maladies rétrovirales contenant un composé comme défini dans la revendication 1 ou 3 ou une combination comme défini dans la revendication 4 ou 5 conjointement avec au moins un véhicule pharmaceutiquement acceptable, excipient, diluant, cryoprotecteur et/ou lyoprotecteur.

13. Composition pharmaceutique pour l'utilisation selon la revendication 12, qui convient à l'administration orale, parentérale, topique et/ou par inhalation.
